(19) Europäisches Patentamt
European Patent Office
Office européen des brevets

(11) EP 3 495 398 B1

(12) EUROPEAN PATENT SPECIFICATION

(45) Date of publication and mention of the grant of the patent:
**19.07.2023 Bulletin 2023/29**

(21) Application number: **17837080.5**

(22) Date of filing: **04.08.2017**

(51) International Patent Classification (IPC):
***B01D 67/00*** *(2006.01)* ***B01D 69/08*** *(2006.01)*
***B01D 71/68*** *(2006.01)* ***B01D 71/76*** *(2006.01)*
***C08F 220/54*** *(2006.01)* ***C08F 226/02*** *(2006.01)*
***C08F 226/10*** *(2006.01)* *A61M 1/34* *(2006.01)*

(52) Cooperative Patent Classification (CPC):
(C-Sets available)
**B01D 67/0093; B01D 69/08; B01D 71/68; B01D 71/76; C08F 218/04; C08F 220/12; C08F 220/54; C08F 226/02; C08F 226/10;**
A61M 1/34; A61M 1/3673; A61M 2205/7563; B01D 71/28; B01D 71/38; B01D 71/40; (Cont.)

(86) International application number:
**PCT/JP2017/028339**

(87) International publication number:
**WO 2018/025979 (08.02.2018 Gazette 2018/06)**

# (54) COPOLYMER, AS WELL AS SEPARATION MEMBRANE, MEDICAL DEVICE, AND BLOOD PURIFIER IN WHICH SAID COPOLYMER IS USED

COPOLYMER SOWIE TRENNMEMBRAN, MEDIZINISCHE VORRICHTUNG UND BLUTREINIGER MIT DIESEM COPOLYMER

COPOLYMÈRE AINSI QUE MEMBRANE DE SÉPARATION, DISPOSITIF MÉDICAL ET PURIFICATEUR DE SANG DANS LESQUELS LEDIT COPOLYMÈRE EST UTILISÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.08.2016 JP 2016154760**

(43) Date of publication of application:
**12.06.2019 Bulletin 2019/24**

(73) Proprietor: **Toray Industries, Inc.**
**Tokyo 103-8666 (JP)**

(72) Inventors:
- **BABA, Takeshi**
  **Otsu-shi, Shiga 520-8558 (JP)**
- **KAWAKAMI, Tomonori**
  **Otsu-shi, Shiga 520-8558 (JP)**
- **USHIRO, Suguru**
  **Otsu-shi, Shiga 520-8558 (JP)**
- **UENO, Yoshiyuki**
  **Otsu-shi, Shiga 520-8558 (JP)**

(74) Representative: **Kador & Partner Part mbB**
**Corneliusstraße 15**
**80469 München (DE)**

(56) References cited:
**WO-A1-2012/176841 WO-A1-2016/158388**
**WO-A1-2016/158388 JP-A- H03 286 774**
**JP-A- S51 137 677 JP-A- 2006 518 420**

- **M. TIJINK ET AL: "Development of novel membranes for blood purification therapies based on copolymers of N-vinylpyrrolidone and n-butylmethacrylate", JOURNAL OF MATERIALS CHEMISTRY B, vol. 1, no. 44, 10 July 2013 (2013-07-10), pages 6066-6076, XP055561240, GB ISSN: 2050-750X, DOI: 10.1039/c3tb20964d**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **TIJINK,M. et al.: "Development of novel membranes for blood purification therapies based on copolymers of N-vinylpyrrolidone and n-butylmethacrylate", Journal of Materials Chemistry B, vol. 1, no. 44, 2013, pages 6066-6077, XP055561240, ISSN: 2050-7518**

(52) Cooperative Patent Classification (CPC): (Cont.)
B01D 71/44; B01D 2325/36; B01D 2325/38

C-Sets
C08F 220/54, C08F 220/1802;
C08F 226/02, C08F 218/10;
C08F 226/10, C08F 218/08;
C08F 226/10, C08F 218/10

## Description

### BACKGROUND OF THE INVENTION

#### Field of the Invention

The present invention relates to a copolymer, and a separation membrane, a medical device, and a blood purifier using the copolymer.

#### Background Art

In a separation membrane for medical use that comes into contact with a body fluid or blood, adhesion of proteins and platelets becomes a cause for a decrease in performance of the separation membrane or for producing a biological reaction, which has been a serious problem.

Patent Document 1 discloses a polysulfone-based polymer obtained by a method in which polyvinylpyrrolidone, which is a hydrophilic polymer, is mixed at a stage of a membrane-forming stock solution and the resultant mixture is molded so that hydrophilicity is imparted to the membrane and contamination is suppressed.

Patent Document 2 discloses a method for coating polyvinylacetal diethylaminoacetate and a hydrophilizing agent on a membrane to attempt to perform hydrophilization.

Patent Document 3 reports a method for bringing a separation membrane of a polysulfone-based polymer into contact with a solution of a hydrophilic polymer such as polyvinylpyrrolidone, and then forming a coating layer insolubilized by radiation crosslinking, while Non Patent Document 1 reports that adhesion of proteins, etc. can only be temporarily suppressed.

Patent Document 4 discloses a separation membrane of a polysulfone-based polymer in which a vinylpyrrolidone/vinyl acetate copolymer is introduced onto the surface. Furthermore, Non Patent Document 2 discloses membranes for blood purification based on copolymers of N-vinylpyrrolidone and n-butyl methacrylate; having good blood compatibility and low platelet adhesion.

#### Prior Art Document

[Patent Document]

[Patent Document 1] JPH 2-18695 B
[Patent Document 2] JPH 8-131791 A
[Patent Document 3] JPH 6-238139 A
[Patent Document 4] JP 2011-72987 A

[Non-Patent Document]

[Non Patent Document 1] Kazunori Kataoka et al., Nano Bioengineering, Kyorin Tosho, 1st edition issued in October 2007, p.115-116
[Non Patent Document 2] M. Tijink et al., Journal of materials chemistry B, vol. 1, no. 44, 10 July 2013 (2013-07-10), p. 6066-6076

### SUMMARY OF THE INVENTION

However, to impart hydrophilicity to the surface of the polysulfone-based polymer mentioned in Patent Document 1, a large amount of a hydrophilic polymer in a membrane-forming stock solution need to be used, and there was a limitation that the hydrophilic polymer imparted to the surface of the polysulfone-based polymer is limited to a hydrophilic polymer compatible with the base polymer.

In the method mentioned in Patent Document 2, there is a concern that polyvinylacetal diethylaminoacetate covers a hydrophilizing agent, resulting in a sharp decrease in the effect on non-adhesion. The present situation is that when a membrane is immersed in each solution of polyvinylacetal diethylaminoacetate and a hydrophilizing agent, the separation performance of the membrane is also decreased.

In the methods mentioned in Patent Document 3 and Patent Document 4, when a hydrophilic polymer substance insolubilized in water is used for a medical device that is used in contact with a biological component such as blood for a long period of time, like a continuous blood purifier, blood coagulation and protein adhesion progress with time due to the contact with the biological component such as blood, eventually leading to clogging, and long-term continuous use is difficult. For example, a blood purifier poses a problem that adhesion of proteins to a membrane in the blood purifier and blood coagulation progress with time, and particularly in a continuous blood purifier used for treatment of acute renal failure, continuous use for one to several days is required, and thus it is imperative to make a specification in which adhesion of proteins and platelets is suppressed and high water permeability can be maintained.

Accordingly, an object of the present invention is to provide a copolymer that maintains high water permeability even when brought into contact with a biological component such as proteins and blood for a long period of time and that suppresses adhesion of proteins and platelets.

As mentioned above, when the surface of a separation membrane is covered with a hydrophilic polymer such as polyvinylpyrrolidone, a sufficient effect to suppress adhesion of proteins, etc. for a long period of time is not obtained, clogging occurs, and continuous use of the separation membrane becomes impossible. This is considered to be because when a polymer existing on a contact surface of a separation membrane of a medical device is too hydrophilic, the polymer and absorbed water of the polymer destabilize the structures of a protein and absorbed water of the protein, so that adhesion of proteins cannot be sufficiently suppressed. Herein, the absorbed water means a water molecule existing near a copolymer existing on a contact surface of a material, or a water molecule existing near a protein.

The present inventors conducted intensive studies to solve the above problems, and as a result, the present inventors found that a copolymer including two or more types of monomer units and the hydration energy density of the copolymer and the monomer unit constituting the copolymer are important for designing a polymer that can suppress adhesion of proteins and platelets, and found that the following copolymer, and separation membrane, medical device, and blood purifier using the copolymer.

The copolymer of the present invention, as defined in the appended claims, can suppress adhesion of proteins and platelets and can maintain high water permeability even when used in contact with a biological component such as blood for a long period of time, and thus is highly useful as a separation membrane and particularly can be used as a medical device and a blood purifier.

## BRIEF DESCRIPTION OF THE DRAWINGS

Fig. 1 is a schematic diagram showing a circuit used for measuring the temporal change of a sieving coefficient of albumin.

## DETAILED DESCRIPTION OF THE INVENTION

The present invention will be described in detail below, but the present invention is not limited to the following embodiments. The ratio of the drawing is not always consistent with that of the description.

The copolymer of the present invention is a copolymer, as defined in the appended claims, comprising two or more types of monomer units, the two or more types of monomer units comprising a hydrophobic monomer unit and a hydrophilic monomer unit, wherein a hydration energy density of the copolymer calculated based on the abovementioned Formula (1) is 167.360 to 209.200 $kJ \cdot ml^{-1} \cdot nm^{-3}$, a monomer unit with a highest hydration energy density of monomer unit i calculated based on the abovementioned Formula (2) is a monomer unit not containing a hydroxy group, a volume fraction of a monomer unit with a highest hydration energy density of monomer unit i calculated based on the abovementioned Formula (3) is 35 to 90%, and a difference in hydration energy density calculated by the following Formula (4) is 71.128 to 418.400 $kJ \cdot mol^{-1} \cdot nm^{-3}$. Herein, 158.992 to 209.200 $kJ \cdot mol^{-1} \cdot nm^{-3}$ is synonymous with 38 to 50 $cal \cdot mol^{-1} \cdot Å^{-3}$ , and 71.128 to 418.400 $kJ \cdot mol^{-1} \cdot nm^{-3}$ is synonymous with 17 to 100 $cal \cdot mol^{-1} \cdot Å^{-3}$. In this regard, 1 cal was defined as 4.184 J.

"Monomer unit" refers to a repeating unit in a homopolymer or a copolymer obtained by polymerizing monomers. For example, hydrophobic monomer unit refers to a repeating unit in a homopolymer or a copolymer obtained by polymerizing hydrophobic monomers.

"Comprising two or more types of monomer units" means that two or more types of repeating units in a copolymer obtained by polymerizing monomers are included. For example, a vinylpyrrolidone/vinyl decanoate random copolymer includes two types of monomer units of vinylpyrrolidone and vinyl decanoate.

"Copolymer" means a polymer composed of two or more types of monomer units.

"Hydration energy" means an energy change obtained in a system when a solute is put in an aqueous solution. As the unit of hydration energy, for example, $cal \cdot mol^{-1}$ or $J \cdot mol^{-1}$ is used.

"Hydration energy of monomer unit" means an absolute value of a value obtained by subtracting energy in vacuum of a monomer unit from energy in water of the monomer unit.

"Hydration energy density" means hydration energy per unit volume. For example, in the case of monomer, it is a numerical value defined by the abovementioned Formula (2). The unit of hydration energy density depends on the unit of hydration energy, and, for example, $cal \cdot mol^{-1} \cdot Å^{-3}$ or $kJ \cdot mol^{-1} \cdot nm^{-3}$ is used.

"Difference in hydration energy density" means a numerical value defined by the abovementioned Formula (4).

"Monomer unit not containing hydroxy group" means that the structure of the monomer unit does not contain a hydroxy group.

"Monomer unit with highest hydration energy density of monomer unit i" means a monomer unit which has the highest hydration energy density defined by the abovementioned Formula (2) in monomer units i constituting a copolymer.

"Monomer unit with lowest hydration energy density of monomer unit i" means a monomer unit which has the lowest hydration energy density defined by the abovementioned Formula (2) in monomer units i constituting a copolymer.

With respect to the molecular model of the monomer unit, for example, when the monomer unit is a structure represented by the chemical formula of the following Formula (I), a structure represented by the chemical formula of the following Formula (II) is included in calculation. In other words, a structure in which the carbon terminal on a side to which a side chain R is bound is terminated with a methyl group ((a) in the following Formula (II)) and the carbon terminal on a side to which a side chain R is not bound is terminated with a hydrogen atom ((b) in the following Formula (II)) is used.

[Chemical Formula 1]

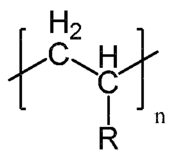

(I)

[Chemical Formula 2]

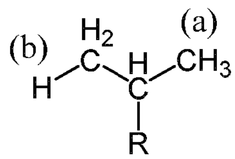

(II)

Energy in vacuum and energy in water of the monomer unit in the abovementioned Formula (1) can be calculated by the following method.

First, structure optimization of a molecular model of the monomer unit is performed. Density functional theory is used for the structure optimization. B3LYP is used for the functional, and 6-31G(d,p) is used for the basis function. In addition, opt is set as a keyword entered in an input file.

Next, energy in vacuum and energy in water are calculated for the optimized structure.

In calculation of energy in vacuum, density functional theory is used. B3LYP is used for the functional, and 6-31G(d,p) is used for the basis function.

In calculation of energy in water, density functional theory is used. B3LYP is used for the functional, and 6-31G(d,p) is used for the basis function. In addition, a polarizable continuum model is used for calculation of energy in water, and the following parameters are used as keywords:

SCRF = (PCM, G03Defaults, Read, Solvent = Water)
Radii = UAHF
Alpha = 1.20

When SCF energy in vacuum and water is calculated, the hydration energy of the monomer unit is determined. Herein, SCF energy is a value of E written in a row of "SCF Done:".

For the energy calculation, quantum chemical calculation software Gaussian09, Revision D.01 (registered trademark) manufactured by Gaussian, Inc. is used.

In the copolymer, the hydration energy density of the copolymer is defined based on the following Formula (1).

$$\text{Hydration energy density (kJ·mol}^{-1}\text{·nm}^{-3}\text{) of copolymer} = \sum_{i=1}^{N}\{(\text{molar fraction of monomer unit i}) \times (\text{hydration energy of monomer unit i})\} / \sum_{i=1}^{N}\{(\text{molar fraction of monomer unit i}) \times (\text{volume of monomer unit i})\} \quad \cdots \text{Formula (1)},$$

wherein a hydration energy of monomer unit i is an absolute value of a value obtained by subtracting energy in vacuum of monomer unit i from energy in water of monomer unit i, N represents a total number of monomer species constituting the copolymer, and i represents an integer of 1 or more and N or less.

The volume of the monomer unit can be calculated using, for example, the Connollysurface method in MaterialsStudio (registered trademark) manufactured by BIOVIA Corp. In that case, the parameters set are as follows:

Gridresolution = Coarse
Gridinterval = 0.75Å (0.075 nm)
vdWfactor = 1.0
Connollyradius = 1.0Å (0.1 nm)

For the volume of the monomer unit in the abovementioned Formula (1), the optimized structure is used.

As the unit of the hydration energy density of the copolymer, for example, cal·mol$^{-1}$·Å$^{-3}$ or kJ·mol$^{-1}$·nm$^{-3}$ is used.

The hydration energy density of the copolymer is 40 to 50 cal·mol$^{-1}$·Å$^{-3}$, preferably 40 to 48 cal·mol$^{-1}$·Å$^{-3}$, more preferably 40 to 45 cal·mol$^{-1}$·Å$^{-3}$, and still more preferably 40 to 44 cal·mol$^{-1}$·Å$^{-3}$. Any preferable lower limit can be combined with any preferable upper limit. In other words, the hydration energy density of the copolymer is 167.360 to 209.200 kJ·mol$^{-1}$·nm$^{-3}$, preferably 167.360 to 200.832 kJ·mol$^{-1}$·nm$^{-3}$, more preferably 167.360 to 188.280 kJ·mol$^{-1}$·nm$^{-3}$, and still more preferably 167.360 to 184.096 kJ·mol$^{-1}$·nm$^{-3}$. Any preferable lower limit can be combined with any preferable upper limit.

The upper limit of the total number N of monomer species constituting the copolymer is not particularly limited, and preferably 2 to 5, more preferably 2 to 3, and most preferably 2.

It is considered that when the hydration energy density of the entire copolymer is out of the abovementioned range, the copolymer and absorbed water of the copolymer destabilize the structures of a protein and absorbed water of the protein. As a result, the electrostatic interaction or the hydrophobic interaction between the copolymer existing on a material surface and a protein causes protein adhesion. Generally, when a polarized functional group such as a carbonyl group (e.g., an ester group and an amide group) is included in a molecule, hydration energy tends to be higher, compared with an alkyl group. The numerical value of hydration energy density becomes higher when the volume of a monomer is lower as long as hydration energy is the same. Therefore, the hydration energy density of the entire copolymer can meet the abovementioned range by adjusting a molar fraction. Examples of the sequence of a hydrophilic monomer unit and a hydrophobic monomer unit in the copolymer include a graft copolymer, a block copolymer, an alternating copolymer, and a random copolymer. Among them, a block copolymer, an alternating copolymer, and a random copolymer are preferred from the viewpoint of a high protein and platelet adhesion suppressing function, and a random copolymer or an alternating copolymer is more preferred from the viewpoint of an appropriate balance between hydrophilicity and hydrophobicity in one molecule. A copolymer in which at least a part of monomer sequences are randomly arranged is regarded a random copolymer.

In the monomer unit, a monomer unit with a highest hydration energy density calculated based on the following Formula (2) is a monomer unit not containing a hydroxy group. It is known that use of regenerated cellulose, which is a material first developed as a blood permeable membrane material, causes transient leukopenia (Hidemune Naito, Biocompatibility of Dialytic Membrane, Tokyo Igakusha Ltd., 1st edition issued on March 25, 2010, p.19). This is because a hydroxy group possessed by regenerated cellulose activates a complement system. To prevent such phenomenon, the monomer unit shall be a monomer unit not containing a hydroxy group.

$$\text{Hydration energy density (kJ·mol}^{-1}\text{·nm}^{-3}\text{) of monomer unit i} = (\text{hydration energy of monomer unit i})/(\text{volume of monomer unit i}) \quad \cdots \text{Formula (2)}$$

In the present invention, the molar fraction of the abovementioned Formula (1) and the following Formula (3) is calculated from the peak area measured with a nuclear magnetic resonance (NMR) apparatus as mentioned later. When the molar fraction cannot be calculated by NMR measurement for reasons such as overlap of peaks, the molar fraction may be calculated by elemental analysis.

$$\text{Volume fraction (\%) of monomer unit with highest hydration energy density of monomer unit i} = \text{molar fraction of monomer unit with highest hydration energy density of monomer unit i} \times \text{volume of monomer unit with highest hydration energy density of monomer unit i} / \sum_{i=1}^{N} \{(\text{molar fraction of monomer unit i}) \times (\text{volume of monomer unit i})\} \quad \cdots \text{Formula (3)},$$

wherein N and i are the same as defined above.

"Biological component" means a substance containing proteins, lipids, and carbohydrates possessed by a living body, in addition to blood and body fluids constituting the living body, and among them, blood is preferable as the subject.

If the number average molecular weight of the copolymer is too low, the effect may not be sufficiently exerted and adhesion of proteins and platelets may become difficult to suppress when the copolymer is introduced onto a material surface. Thus, the number average molecular weight is preferably 2,000 or more, and more preferably 3,000 or more. On the other hand, the upper limit of the number average molecular weight of the copolymer is not particularly limited, but the number average molecular weight is preferably 1,000,000 or less, more preferably 100,000 or less, and still more preferably 50,000 or less since the efficiency of introduction onto a material surface may decrease if the number average molecular weight is too high.

In the copolymer, the volume fraction of the monomer unit with the highest hydration energy density of monomer unit i calculated based on the abovementioned Formula (3) is 35% to 90%, preferably 40% to 80%, more preferably 40% to 75%, and still more preferably 40% to 70%. Any preferable lower limit can be combined with any preferable upper limit.

It is considered that when the volume fraction is in the abovementioned range, both effects of a hydrophilic monomer unit and a hydrophobic monomer unit lead to appropriate magnitude of interaction by the copolymer existing on a material surface and absorbed water of the copolymer to a protein and absorbed water of the protein, resulting in suppression of adhesion of proteins.

In the copolymer, the difference in hydration energy density is calculated by the following Formula (4):

$$\text{Difference in hydration energy density } (kJ \cdot mol^{-1} \cdot nm^{-3}) = (\text{hydration energy density of monomer unit with highest hydration energy density of monomer unit}) - (\text{hydration energy density of monomer unit with lowest hydration energy density of monomer unit}) \quad \cdots \text{Formula (4)}.$$

The difference in hydration energy density is 17 to 100 $cal \cdot mol^{-1} \cdot Å^{-3}$, preferably 17 to 75 $cal \cdot mol^{-1} \cdot Å^{-3}$, and more preferably 17 to 60 $cal \cdot mol^{-1} \cdot Å^{-3}$ . Any preferable lower limit can be combined with any preferable upper limit. In other words, the difference in hydration energy density is 71.128 to 418.400 $kJ \cdot mol^{-1} \cdot nm^{-3}$, preferably 71.128 to 313.800 $kJ \cdot mol^{-1} \cdot nm^{-3}$, and more preferably 71.128 to 251.040 $kJ \cdot mol^{-1} \cdot nm^{-3}$. Any preferable lower limit can be combined with any preferable upper limit.

The hydration energy density, the volume fraction, and the difference in hydration energy density may be optionally combined.

It is considered that when the difference in hydration energy density is in the abovementioned range, a hydrophilic monomer unit of a copolymer existing on a material surface can play a role in retaining absorbed water and a hydrophobic monomer unit can play a role in controlling the mobility of absorbed water. As a result, it is considered that the interaction of the copolymer existing on a material surface and absorbed water of the copolymer to a protein and absorbed water of the protein becomes appropriate magnitude, resulting in suppression of adhesion of proteins.

The two or more types of monomer units comprise a hydrophobic monomer unit and a hydrophilic monomer unit.

"Hydrophobic monomer unit" means a monomer unit with a lower hydration energy density than that of a hydrophilic monomer unit, and, a repeating unit in a copolymer obtained by polymerizing monomers selected from the group consisting of vinyl carboxylate, acrylate, and a styrene derivative is suitably used. Among them, a repeating unit in a copolymer obtained by copolymerizing vinyl carboxylate is more preferable, and a repeating unit in a homopolymer obtained by polymerizing vinyl carboxylate is more preferable since a balance with a hydrophilic monomer unit and the mobility of absorbed water existing on a material surface is easily controlled.

The vinyl carboxylate means vinyl carboxylate ester, and examples thereof include aromatic vinyl carboxylate

and aliphatic vinyl carboxylate. Examples of the aromatic vinyl carboxylate include vinyl benzoate, vinyl alkylbenzoate, vinyl oxybenzoate, and vinyl chlorbenzoate, but the aromatic vinyl carboxylate is not particularly limited. Examples of the aliphatic vinyl carboxylate include saturated vinyl carboxylates such as vinyl acetate, vinyl propionate, vinyl butyrate, vinyl valerate, vinyl caproate, vinyl laurate or vinyl palmitate, and unsaturated vinyl carboxylates such as vinyl acrylate, vinyl methacrylate, vinyl crotonate or vinyl sorbate, and the aliphatic vinyl carboxylate is not particularly limited. These aromatic vinyl carboxylates or aliphatic vinyl carboxylates may have a substituent as long as it does not impair the object of the invention.

"Hydrophilic monomer unit" means a monomer unit with higher hydration energy density than that of a hydrophobic monomer unit, and, a repeating unit in a copolymer obtained by polymerizing monomers selected from the group consisting of an allylamine derivative, a vinylamine derivative, N-vinylamide, an acrylamide derivative, a methacrylamide derivative, and N-acryloylmorpholine is suitably used.

The allylamine derivative means an organic compound having an allyl group ($CH_2$=CH-$CH_2$-) and an amino group (-$NH_2$, -NH, or -N), and examples of the allylamine derivative include allylamine, N-methylallylamine, N-isopropylallylamine, and N-tert-butylallylamine. The allylamine derivative may have a substituent as long as it does not impair the invention.

The vinylamine derivative means an organic compound having a vinylamine structure ($CH_2$=CH-NH-), and examples of the vinylamine derivative include vinylamine and vinylhydrazine. The vinylamine derivative may have a substituent as long as it does not impair the invention.

The N-vinylamide means an organic compound having an N-vinylamide structure ($CH_2$=CH-NH-CO-), and examples thereof include N-vinylcarboxylic acid amide. Examples of the N-vinylcarboxylic acid amide include N-vinylacetamide, N-vinylpropionamide, N-vinylbutyric acid amide, and N-vinylbenzamide. The N-vinylamide may have a substituent as long as it does not impair the invention.

The acrylamide derivative means an organic compound having an acrylamide structure ($CH_2$=CH-CO-NH-), and examples of the acrylamide derivative include acrylamide, N-isopropylacrylamide, N-tert-butylacrylamide, and N-phenylacrylamide. The acrylamide derivative may have a substituent as long as it does not impair the invention.

The methacrylamide derivative means an organic compound having a methacrylamide structure ($CH_2$=C($CH_3$)-CO-NH-), and examples of the methacrylamide derivative include methacrylamide, N-isopropylmethacrylamide, and N-phenylmethacrylamide. The methacrylamide derivative may have a substituent as long as it does not impair the invention.

The hydrophobic monomer unit and the hydrophilic monomer unit are combined. Examples of the combination include vinyl carboxylate and N-vinylamide, and acrylate and an acrylamide derivative, and the like. As long as the action and function of the copolymer are not impaired, a different monomer, for example, a monomer including a reactive group such as a glycidyl group may be copolymerized.

Examples of the sequence of a hydrophilic monomer unit and a hydrophobic monomer unit in the copolymer include a graft copolymer, a block copolymer, an alternating copolymer, and a random copolymer. Among them, a block copolymer, an alternating copolymer, and a random copolymer are preferred from the viewpoint of a high protein and platelet adhesion suppressing function, and a random copolymer or an alternating copolymer is more preferred from the viewpoint of an appropriate balance between hydrophilicity and hydrophobicity in one molecule. The reason why a block copolymer, an alternating copolymer, and a random copolymer have a higher protein and platelet adhesion suppressing function than that of a graft copolymer, for example, a graft copolymer having a main chain composed of a hydrophilic monomer unit and a side chain composed of a hydrophobic monomer unit, is considered as follows. In the graft copolymer, since the monomer unit portion grafted to the main chain has many opportunities to come into contact with proteins or the like, the properties of the graft chain portion have a greater influence than the properties of the copolymerized polymer. The reason why the alternating copolymer and the random copolymer are more preferred in view of an appropriate balance between hydrophilicity and hydrophobicity than the block copolymer is considered that the properties of each monomer unit are clearly divided in the block copolymer.

The copolymer can be synthesized, for example, by a chain polymerization method typified by a radical polymerization method using an azo type initiator, but the synthesis method is not limited thereto.

The copolymer is manufactured by the following manufacturing method, but the method is not limited thereto.

Each predetermined amount of a hydrophilic monomer and a hydrophobic monomer and a polymerization solvent and a polymerization initiator are mixed under stirring at a predetermined temperature for a predetermined period of time in a nitrogen atmosphere to cause a polymerization reaction. The quantitative ratio between the hydrophilic monomer and the hydrophobic monomer can be determined according to the molar fraction of the hydrophilic monomer unit in the copolymer. The reaction liquid is cooled to room temperature to stop the polymerization reaction, and the liquid is charged into a solvent such as hexane. The deposited precipitate is collected and dried under reduced pressure to obtain a copolymer.

The reaction temperature of the polymerization reaction is preferably 30 to 150°C, more preferably 50 to 100°C, and still more preferably 70 to 80°C.

The pressure of the polymerization reaction is preferably normal pressure.

The reaction time of the polymerization reaction is appropriately selected according to conditions such as reaction temperature, and is preferably 1 hour or more, more preferably 3 hours or more, and still more preferably 5 hours or more. If the reaction time is short, a large amount of unreacted monomers may tend to remain in the copolymer. On the other hand, the reaction time is preferably 24 hours or less and more preferably 12 hours or less. If the reaction time is long, side reactions such as formation of dimers tend to occur, which may make it difficult to control the molecular weight.

The polymerization solvent used for the polymerization reaction is not particularly limited as long as it is a solvent compatible with the monomers. For example, ether-based solvents such as dioxane or tetrahydrofuran, amide-based solvents such as N,N-dimethylformamide, sulfoxide-based solvents such as dimethylsulfoxide, aromatic hydrocarbon-based solvents such as benzene or toluene, alcohol-based solvents such as methanol, ethanol, isopropyl alcohol, amyl alcohol, or hexanol, or water, or the like is/are used. From the viewpoint of toxicity, alcohol-based solvents or water is/are preferably used.

As the polymerization initiator for the polymerization reaction, for example, a photopolymerization initiator or a thermal polymerization initiator is used. A polymerization initiator that generates any of a radical, a cation or an anion may be used, but a radical polymerization initiator is suitably used from the viewpoint that it does not cause decomposition of the monomers. Examples of the radical polymerization initiator include azo type initiators such as azobisisobutyronitrile, azobisdimethylvaleronitrile, or dimethyl azobis(isobutyrate), or peroxide initiators such as hydrogen peroxide, benzoyl peroxide, di-tert-butyl peroxide, or dicumyl peroxide.

The solvent into which the polymerization reaction solution is charged after stopping of the polymerization reaction is not particularly limited as long as it is a solvent in which the copolymer precipitates. For example, hydrocarbon-based solvents such as pentane, hexane, heptane, octane, nonane, or decane, or ether-based solvents such as dimethyl ether, ethyl methyl ether, diethyl ether, or diphenyl ether are used.

The copolymer is suitably used for a separation membrane from the viewpoint that it can suppress adhesion of proteins and platelets and can maintain water permeability even when used in contact with a biological component such as blood for a long period of time.

The present invention is characterized by providing a separation membrane including the copolymer and a medical device including the copolymer.

"Separation membrane" means a membrane that selectively removes certain substances contained in a liquid to be treated, such as blood or an aqueous solution, by adsorption or based on the size of the substances, and examples thereof include an ultrafiltration membrane and a reverse osmosis membrane. In the separation membrane, suppression of adhesion of proteins is required, and achievement of this is preferable for a medical device incorporating the separation membrane. The copolymer is preferably introduced onto the surface of the separation membrane. The form of the separation membrane includes a flat membrane and a hollow fiber membrane, and the hollow fiber membrane means a pipe-like shaped separation membrane.

"Medical device" is mainly used in contact with a biological component such as blood or a body fluid. Specific examples of the medical device include a blood purifier, a plasma separator, an artificial organ, a blood circuit, a blood storage bag, a catheter, or a stent, and among them, a blood purifier is preferable. A blood purifier, an artificial organ, or the like is an example of a medical device using a separation membrane module. To suppress adhesion of proteins and platelets, the copolymer can prevent formation of a thrombus by being used for a medical device such as a catheter and a stent. In a medical device, the copolymer is more preferably introduced onto a surface in contact with a biological component such as blood, and in the case of a catheter, a stent, or the like, the copolymer is preferably introduced onto a surface of a (metal) material in contact with a biological component such as mainly blood. In the case of a blood circuit, the copolymer is preferably introduced onto an inner surface in contact with a biological component such as mainly blood in a tube, etc., constituting the circuit.

Herein, "blood purifier" refers to a medical device incorporating a separation membrane having a function of circulating blood out of the body to remove waste products and harmful substances in blood, and examples thereof include an artificial kidney module and an exotoxin adsorption column. The copolymer is preferably introduced onto a surface of a separation membrane to be incorporated.

Although there are various forms of utilization of the copolymer, for example, in the case of a separation membrane including the copolymer, it is necessary to introduce the copolymer onto at least a part of a surface on a side in contact with a biological component such as blood among surfaces of the separation membrane. Although it is possible to prepare a separation membrane using the copolymer itself, it is more preferable to introduce the copolymer onto another material surface from the viewpoint of the strength of the separation membrane.

For example, immersing a flat membrane of polyethylene terephthalate used in an artificial blood vessel or the like in an aqueous solution of the copolymer can suppress adhesion of platelets. From the viewpoint of preventing formation of a thrombus at the membrane surface, the number of adhered platelets per an area of $4.3 \times 10^3$ $\mu m^2$ is preferably 20 or less, more preferably 10 or less, still more preferably 5 or less, and yet more preferably 0 or less. The

concentration of the aqueous solution of the copolymer is preferably 0.01 ppm or more, and more preferably 0.1 ppm or more. The number of adhered platelets is measured by the method described later.

Moreover, the copolymer as a component for forming the separation membrane may be introduced onto the surface of the membrane (in particular, the inner surface which is often brought into contact with blood) to suppress the adhesion of blood components, and the separation membrane may be incorporated into a casing and used as a separation membrane module for medical use. The form of the separation membrane is preferably a hollow fiber membrane, and preferably a hollow fiber membrane module in which the hollow fiber membrane is incorporated into a casing.

"Introduce a copolymer onto a surface" means to place (coating, chemical bonding, or the like) the copolymer on a material surface by a method such as coating or immersion. For example, in the case of a separation membrane, a method for forming a membrane and then coating a copolymer is preferable, and a method for bringing the copolymer as a solution (preferably an aqueous solution) into contact with the surface of the membrane is preferably used. More specifically, there can be mentioned a method for flowing a solution of the copolymer at a predetermined flow rate, and a method for immersing the membrane in the solution. In addition, in a method for adding a copolymer to a stock solution for forming a membrane and spinning the stock solution, there is also a method for intentionally setting conditions so that the copolymer gathers on the membrane surface.

Furthermore, as a method for introducing the copolymer onto a material surface, covalent bonding by chemical reaction may be utilized. Specifically, it is achieved by reacting a reactive group on the surface of the base of the material, such as an amino group, a sulfonic acid group, or a halogenated alkyl group with a reactive group introduced into a main chain terminal or a side chain of the copolymer.

Examples of the method for introducing a reactive group onto a material surface include a method for polymerizing monomers having a reactive group to obtain a base having a reactive group on the surface, and a method for introducing a reactive group by ozone treatment or plasma treatment after polymerization.

Examples of the method for introducing a reactive group into the main chain terminal of the copolymer include a method for using an initiator having a reactive group, such as 2,2'-azobis[2-methyl-N-(2-hydroxyethyl)propionamide] or 4,4'-azobis(4-cyanovaleric acid).

Examples of the method for introducing a reactive group into the side chain of the copolymer include a method for copolymerizing monomers having a reactive group, such as glycidyl methacrylate, as long as the action and function of the copolymer are not impaired.

The polymer which can serve as a material of the medical device is not particularly limited, and examples thereof include a polysulfone-based polymer, polystyrene, polyurethane, polyethylene, polypropylene, polycarbonate, polyvinylidene fluoride, polyacrylonitrile, polymethyl methacrylate, polyvinyl chloride, polyamide, polyimide, or polyester. Among them, a polysulfone-based polymer and polymethyl methacrylate are suitably used because they are easy to form a hollow fiber membrane and are easy to be coated with the polymer.

The main raw material of the hollow fiber membrane is preferably a polysulfone-based polymer. Herein, the polysulfone-based polymer is a polymer having an aromatic ring, a sulfonyl group, and an ether group in the main chain, and examples thereof include polysulfone, polyethersulfone, or polyarylethersulfone. Herein, the main raw material represents a raw material contained in an amount of 90% by weight or more based on the entire polysulfone-based polymer.

As the main raw material of the hollow fiber membrane in the present invention, for example, a polysulfone-based polymer represented by the following chemical Formulas (III) and/or (IV) is suitably used, but the main raw material is not limited thereto. In the formulas, n is an integer of 1 or more, and preferably 50 to 80. When n has a distribution, the average value is regarded as n.

[Chemical Formula 3]

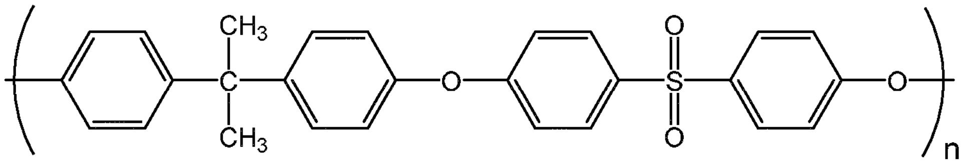

(III)

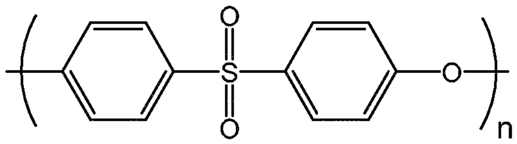

(IV)

The polysulfone-based polymer that can be used in the separation membrane module for medical use is suitably a polymer composed only of the repeating units represented by the abovementioned Formula (III) and/or (IV), but the polysulfone-based polymer may be a copolymer with a different monomer or may be a modified product as long as the effect is not hindered. When the polysulfone-based polymer is copolymerized with a different monomer, the copolymerize ratio of the different monomer is preferably 10% by weight or less based on the entire polysulfone-based polymer.

Specific examples of the polysulfone-based polymer that can be used in the separation membrane module for medical use include polysulfone-based polymers such as Udel Polysulfone P-1700 and P-3500 (manufactured by SOLVAY), Ultrason (registered trademark) S3010 and S6010 (manufactured by BASF Corporation), VICTREX (manufactured by Sumitomo Chemical Company, Limited), Radel (registered trademark) A (manufactured by SOLVAY), and Ultrason (registered trademark) E (manufactured by BASF Corporation).

As a method for manufacturing the separation membrane module for medical use, there are various methods according to the use thereof. As one aspect thereof, the manufacturing method can be divided into a step of manufacturing a separation membrane and a step of incorporating the separation membrane into a module. Furthermore, in manufacturing the separation membrane module, a treatment by radiation irradiation may be used before the step of incorporating the separation membrane into a module, or after the step of incorporating the separation membrane into a module. Performing a treatment by irradiation with $\gamma$-rays as a treatment by radiation irradiation after the step of incorporating the separation membrane into a module is preferred in that sterilization can be performed at the same time because the separation membrane module is intended for medical use.

The separation membrane module for medical use used in a blood purifier is preferably a hollow fiber membrane module, and one example of a method for manufacturing the same will be described.

An example of a method for manufacturing a hollow fiber membrane incorporated into a blood purifier is the following method. That is, a stock solution (the concentration of polysulfone and polyvinylpyrrolidone is preferably 10 to 30% by weight, and more preferably 15 to 25% by weight) obtained by dissolving polysulfone and polyvinylpyrrolidone (the weight ratio is preferably 20: 1 to 1:5, and more preferably 5: 1 to 1:1) in a mixed solution of a good solvent for polysulfone (preferably N,N-dimethylacetamide, dimethylsulfoxide, N,N-dimethylformamide, N-methylpyrrolidone, or dioxane, or the like) and a poor solvent therefor (e.g., water, glycerin, or the like) is discharged from a double annular spinneret while flowing an injection solution through the inside of the spinneret, and the stock solution and the injection solution are let to travel in a dry part and then led to a coagulation bath. At this time, since the humidity of the dry part has some influence, it is also possible to accelerate the phase separation behavior near the outer surface of the membrane by moisture supply from the outer surface during traveling of the membrane in the dry part to increase the pore diameter, and consequently reduce the permeation/diffusion resistance during the dialysis. However, if the relative humidity is too high, the coagulation of the stock solution on the outer surface becomes dominant and the pore diameter rather decreases, which consequently tends to increase the permeation/diffusion resistance during the dialysis. Therefore, the relative humidity is suitably 60 to 90%. As for the composition of the injection solution, it is preferred to use a solution having a composition based on the solvent used for the stock solution from the viewpoint of process suitability. As for the concentration of the injection solution, for example, when N,N-dimethylacetamide is used as the injection solution, an aqueous solution having a concentration of 45 to 80% by weight is suitably used, and an aqueous solution having a concentration of 60 to 75% by weight is more suitably used.

Herein, the good solvent means a solvent in which a subject polymer is dissolved in an amount of 10% by weight or more at 20°C. The poor solvent means a solvent in which a subject polymer is dissolved in an amount of less

than 10% by weight at 20°C.

The method for incorporating the hollow fiber membrane is not particularly limited, and the following method is exemplified. First, the hollow fiber membrane is cut into a required length, required number of the membranes are bundled, and the bundle is placed in a cylindrical case. Then, the case is temporarily capped at both ends, and a potting agent is placed at both ends of the hollow fiber membrane. In this case, a method for placing a potting agent while rotating the module with a centrifuge is preferred because the potting agent is uniformly filled. After the potting agent solidifies, both the ends of the hollow fiber membrane are cut so as to be opened to obtain a hollow fiber membrane module in which the hollow fiber membrane is incorporated into a module.

Since the polysulfone-based polymer used as a main raw material of the hollow fiber membrane is generally strongly hydrophobic, organic substances such as proteins are likely to adhere when the polymer is used as it is as a hollow fiber membrane. Therefore, in the separation membrane module for medical use, a hollow fiber membrane including the copolymer introduced onto the surface is suitably used. Examples of the method for introducing the copolymer onto the surface include a method for bringing a solution in which the copolymer is dissolved into contact with a hollow fiber membrane in the module, and a method for bringing an injection solution containing the copolymer into contact with the inside of the hollow fiber membrane during spinning of the hollow fiber membrane.

When an aqueous solution in which the copolymer is dissolved is passed through a hollow fiber membrane in a module to introduce onto the surface, a sufficient amount of the copolymer is not introduced onto the surface if the copolymer concentration of the aqueous solution is too low. Therefore, the copolymer concentration in the aqueous solution is preferably 10 ppm or more, more preferably 100 ppm or more, and most preferably 300 ppm or more. However, if the copolymer concentration in the aqueous solution is too high, there is a concern that the amount of eluate from the module will increase. Therefore, the copolymer concentration in the aqueous solution is preferably 100,000 ppm or less, more preferably 10,000 ppm or less. The number average molecular weight of the copolymer is measured by gel permeation chromatography (GPC) as mentioned later.

When the copolymer is hardly soluble or insoluble in water, the copolymer may be dissolved in an organic solvent which does not dissolve the hollow fiber, or a mixed solvent of water and an organic solvent which is compatible with water and does not dissolve the hollow fiber. Specific examples of the organic solvent or the organic solvent that can be used in the mixed solvent include, but are not limited to, alcohol-based solvents such as methanol, ethanol, or propanol.

In addition, if the ratio of the organic solvent in the mixed solvent is large, the hollow fiber swells, the copolymer diffuses into the hollow fiber membrane, and it may become difficult to introduce the copolymer efficiently only onto the surface. Therefore, the weight fraction of the organic solvent in the mixed solvent is preferably 60% or less, more preferably 10% or less, and most preferably 1% or less.

In the separation membrane module for medical use, in order to prevent elution of the introduced copolymer at the time of use of the module, it is preferred that the copolymer is insolubilized by radiation irradiation or heat treatment after being introduced onto the surface of the separation membrane.

For the radiation irradiation, α-rays, β-rays, γ-rays, X-rays, ultraviolet rays, or electron beams or the like can be used. For blood purifiers such as artificial kidneys, sterilization before shipping is mandatory. For the sterilization, in recent years, a radiation sterilization method using γ-rays or electron beams is often used from the viewpoint of the low residual toxicity and convenience. Therefore, use of the radiation sterilization method in a state where an aqueous solution in which the copolymer is dissolved is in contact with the hollow fiber membrane in the separation membrane module for medical use is preferred because insolubilization of the copolymer can be achieved simultaneously with sterilization.

In the case of simultaneously performing sterilization and reforming of the hollow fiber membrane in the separation membrane module for medical use, the irradiation dose of radiation is preferably 15 kGy or more, more preferably 25 kGy or more. This is because an irradiation dose of 15 kGy or more is effective for sterilizing a blood purification module or the like with γ-rays. The irradiation dose is preferably 100 kGy or less. If the irradiation dose exceeds 100 kGy, three-dimensional crosslinking and decomposition of the ester group moiety of the vinyl carboxylate monomer unit are likely to occur in the copolymer, which may lower blood compatibility.

In order to suppress the crosslinking reaction upon irradiation with radiation, an antioxidant may be used. An antioxidant means a substance having a property of easily giving electrons to other molecules. Examples thereof include, but are not limited to, water-soluble vitamins such as vitamin C, polyphenols, or alcohol-based solvents such as methanol, ethanol, or propanol. These antioxidants may be used alone or two or more antioxidants may be used in combination. In the case of using the antioxidant in the separation membrane module for medical use, safety should be considered. Therefore, an antioxidant with low toxicity, such as ethanol or propanol, is suitably used.

When the copolymer is introduced onto the surface of the hollow fiber membrane, the amount of the copolymer introduced onto the surface of the hollow fiber membrane can be quantified by attenuated total reflection infrared spectroscopy (ATR-IR) as mentioned later. Furthermore, if necessary, the amount can be quantified also by X-ray photoelectron spectroscopy (XPS) or the like. Herein, the surface of the hollow fiber membrane means the inner surface of

the hollow fiber membrane that comes into contact with blood.

In the present invention, when quantifying the amount of the copolymer introduced onto the surface of the separation membrane by ATR-IR, a ratio of the infrared absorption peak area ($A_{C=O}$) derived from the ester group C=O in the range of 1,711 to 1,751 $cm^{-1}$ to the infrared absorption peak area ($A_{C=C}$) derived from the benzene ring C=C of polysulfone in the range of 1,549 to 1,620 $cm^{-1}$, that is, $(A_{C=O})/(A_{C=C})$ is calculated at three different positions on the membrane surface. Measurement is made at arbitrary three positions in one hollow fiber membrane, the area ratio is calculated, and the average value thereof is regarded as the surface introduction amount of the copolymer. The ATR-IR is capable of measuring the surface up to several micrometers in depth.

In order to sufficiently suppress adhesion of proteins and platelets to the separation membrane module for medical use, the amount of the copolymer introduced onto the surface of the separation membrane is preferably 0.001 or more, more preferably 0.01 or more, and most preferably 0.03 or more. The upper limit of the surface introduction amount of the copolymer is not particularly limited, but if the surface introduction amount of the polymer is too large, the amount of the eluate may increase, and the upper limit is preferably 1.0 or less, more preferably 0.9 or less, and still more preferably 0.8 or less. Any preferable lower limit can be combined with any preferable upper limit.

Examples of the method for quantifying adhesion of proteins and platelets include a method for measuring the reduction rate of water permeability, the amount of adhered platelets, and the temporal change of the sieving coefficient of albumin when bovine blood is perfused into a separation membrane module for medical use in which the copolymer is introduced.

The reduction rate of water permeability is calculated by measuring the water permeability before and after bovine blood is perfused into a separation membrane module for medical use in which the copolymer is introduced onto the surface. Adhesion of proteins and platelets causes clogging of the pores of the hollow fibers, so that the water permeability reduces. Specific procedures are as follows. First, a circuit is connected to an inlet and an outlet on a B side (blood side) of the hollow fiber membrane module, and washed with water at a rate of 200 mL/min for 5 minutes. Next, water (37°C) is flowed at a rate of 200 mL/min, the outflow from the B outlet is adjusted, and a filtration amount V per 1 minute of outflow to a D side and an average pressure P of the B side inlet and outlet are measured. By changing outflow from the B outlet, measurement is performed at three points, and the average value of the value calculated by the following formula is regarded as water permeability [UFRP-0].

$$\text{UFRP (mL/hr/mmHg/m}^2\text{)} = V \times 60/P/A$$

V: Filtration amount (mL/min), P: Pressure (mmHg), A: Membrane area (m2)

Next, 2 L of bovine whole blood is circulated. A hollow fiber membrane module (1) and a blood circuit are connected as shown in Fig. 1. Bovine blood supplemented with heparin is adjusted so that the hematocrit is 30% and the total protein concentration is 6 to 7 g/dl, and put in a circulation beaker (4). The circulation beaker (4) containing the bovine blood is kept at 37°C in a warm water bath (9) equipped with a heater (8). An inlet of a Bi circuit (5), an outlet of a Bo circuit (6), and an outlet of an F circuit (7) are placed in the circulation beaker (4) containing 2 L of the bovine blood adjusted as mentioned above, and a Bi pump (2) is started at a circulation flow rate of 100 ml/min. After 60 minutes, the circulation is stopped. Then, the circuit is connected to an inlet and an outlet on a B side (blood side) of the hollow fiber module, and washed with physiological saline at a rate of 200 mL/min for 10 minutes. Furthermore, the circuit is washed with water at a rate of 200 mL/min for 5 minutes, and then water permeability [UFRP-60] is calculated in the same manner as mentioned above.

The reduction rate of water permeability is calculated by the following formula.

$$\text{Reduction rate \%} = ([\text{UFRP-0}] - [\text{UFRP-60}])/[\text{UFRP - 0}] \times 100$$

The reduction rate of water permeability when a separation membrane using the copolymer is used is preferably 15% or less. Furthermore, when a medical device, for example, a blood purifier, can be used for a long period of time, the reduction rate of water permeability is preferably 10% or less.

In order to quantify adhesion of platelets, the amount of adhered human platelets of the hollow fiber membrane is measured. A double-sided tape is attached to a circular plate 18 mmϕ in diameter made of polystyrene, and a hollow fiber membrane irradiated with γ-rays at 25 kGy is fixed thereto. The attached hollow fiber membrane is trimmed to a semi-cylindrical shape with a single-edged blade to expose the inner surface of the hollow fiber membrane. If there is any contaminant, scratch, crease or the like on the inner surface of the hollow fiber, platelets may adhere to that portion and hinder correct evaluation, and thus attention should be paid. The circular plate is attached to a cylindrically cut Falcon (registered trademark) tube (18 mmϕ in diameter, No. 2051) so that the face to which the hollow fiber membrane was attached is inside of the cylinder, and the gap is filled with Parafilm. The inside of this cylindrical tube is washed

with physiological saline, and then the tube is filled with physiological saline. Human venous blood is collected, and heparin is added to the blood immediately after collection so that the concentration will be 50 U/ml. The physiological saline in the cylindrical tube is discharged, and then 1.0 ml of the blood is put in the cylindrical tube within 10 minutes after the blood collection and shaken at 37°C for 1 hour. Then, the hollow fiber membrane is washed with 10 ml of physiological saline, and blood components are fixed with 2.5% glutaraldehyde physiological saline and washed with 20 ml of distilled water. The washed hollow fiber membrane is dried under reduced pressure at 20°C and 0.5 Torr for 10 hours. This hollow fiber membrane is attached to a sample stage of a scanning electron microscope with a double-sided tape. After that, a Pt-Pd thin film is formed on the hollow fiber membrane surface by sputtering to prepare a sample. The inner surface of this hollow fiber membrane sample is observed with a field emission type scanning electron microscope (manufactured by Hitachi, Ltd.; S800) at a magnification of 1500 times, and the number of adhered platelets in one field of view ($4.3 \times 10^3$ $\mu m^2$) is counted. When 50 or more platelets adhered, it is assumed that no platelet adhesion suppression effect is exerted, and the number of adhered platelets is regarded as 50. Since a pool of blood tends to occur at an end portion in the longer direction of the hollow fiber, the average value of the number of adhered platelets in 20 different fields of view near the center of the hollow fiber membrane is regarded as the number of adhered platelets (number/$4.3 \times 10^3$ $\mu m^2$).

The number of adhered platelets of the separation membrane using the copolymer is preferably 20 or less. Furthermore, in order to make it possible to use a medical device, for example, a blood purifier for a long period of time, the number of adhered platelets is most preferably 0.

In blood purifiers such as artificial kidney modules, adhesion of proteins and platelets not only deteriorates fractionation performance but also inhibits blood circulation inside the hollow fibers due to blood coagulation, and extracorporeal circulation cannot be continued in some cases. The adhesion of proteins and platelets occurs particularly remarkably within 60 minutes after contact with blood. Thus, in the present invention, the sieving coefficients of albumin after 10 minutes and 60 minutes from the start of circulation of blood are measured, and the reduction rate is calculated.

The sieving coefficient of albumin is measured as follows. First, a hollow fiber membrane module (1) and a blood circuit are connected as shown in Fig. 1. Bovine blood supplemented with heparin is adjusted so that the hematocrit is 30% and the total protein concentration is 6 to 7 g/dl, and put in a circulation beaker (4). The circulation beaker (4) containing the bovine blood is kept at 37°C in a warm water bath (9) equipped with a heater (8).

An inlet of a Bi circuit (5), an outlet of a Bo circuit (6), and an outlet of an F circuit (7) are placed in the circulation beaker (4) containing 2 L of the bovine blood adjusted as mentioned above, and a Bi pump (2) is started at a circulation flow rate of 100 ml/min.

The Bi circuit (5) represents a flow path of blood which flows out from the circulation beaker (4), flows through the Bi pump (2), and enters a blood side inlet of the hollow fiber membrane module (1). The Bo circuit (6) represents a flow path of blood which flows out from a blood side outlet of the hollow fiber membrane module (1) and enters the circulation beaker (4). The F circuit (7) represents a flow path of blood which flows out from a dialysate side outlet of the hollow fiber membrane module (1), flows through an F pump (3), and enters the circulation beaker (4). The Bi pump (2) represents a pump used for flowing blood through the Bi circuit (5).

Subsequently, the F pump (3) is started at a filtration flow rate of 10 ml/min, and the blood is sampled over time at the inlet of the Bi circuit (5), the outlet of the Bo circuit (6), and the outlet of the F circuit (7). Note that the F pump (3) represents a pump used for flowing blood through the F circuit (7).

The albumin concentration at each elapsed time from the start of the F pump (3) is measured, and the sieving coefficient of albumin (ScAlb) at each elapsed time is calculated according to the following formula.

$$\text{ScAlb}(\%) = \text{CF}/\{0.5 \times (\text{CBi} + \text{CBo})\} \times 100$$

In the abovementioned formula, CF represents the albumin concentration (g/ml) at the outlet of the F circuit (7), CBo represents the albumin concentration (g/ml) at the outlet of the Bo circuit (6), and CBi represents the albumin concentration (g/ml) at the inlet of the Bi circuit (5).

The reduction rate of the sieving coefficient of albumin after a perfusion time of 60 minutes (ScAlb60) to the sieving coefficient of albumin after a perfusion time of 10 minutes (ScAlb10) is calculated according to the following formula.

$$\text{Reduction rate}(\%) = (\text{ScAlb10} - \text{ScAlb60})/\text{ScAlb10} \times 100$$

In the separation membrane module for medical use in which the copolymer is introduced onto the surface, the reduction rate of the sieving coefficient of albumin after a perfusion time of 60 minutes to the sieving coefficient of albumin after a perfusion time of 10 minutes is preferably 25% or less in order to keep using a separation membrane

for 4 hours. Furthermore, in order to keep using a medical device, for example, a blood purifier for 24 hours, the reduction rate is more preferably 10% or less. Furthermore, in order to make it possible to use a blood purifier for 48 hours or more, the reduction rate of the sieving coefficient of albumin is still more preferably 5% or less.

In order to suppress adhesion of platelets and proteins when using as a separation membrane, it is preferable that the reduction rate of water permeability is 15% or less, the number of adhered platelets is 5 or less, and the reduction rate of the sieving coefficient of albumin is 25% or less. Furthermore, in order to suppress adhesion of proteins and platelets for a long period of time, it is more preferable that the reduction rate of water permeability is 10% or less, the number of adhered platelets is 0 or less, and the reduction rate of the sieving coefficient of albumin is 5% or less.

In a thrombus formation test on a PET filter, the PET filter was cut into a 1 cm × 1 cm piece, and placed in a cylindrical container made of polypropylene with a diameter of 1 cm and a depth of 0.8 cm. To this, 1 ml of human blood supplemented with heparin so that the concentration became 50 U/ml was added so that the filter was immersed, and then shaken for 30 minutes. The filter was taken out, and whether a thrombus was formed or not was confirmed. This procedure enables simple evaluation of whether the medical device can maintain antithrombogenicity and can be used for a long period of time.

Since the copolymer can maintain the property of suppressing adhesion of platelets and proteins for a long period of time, it is suitably used in particularly medical devices. In particular, the copolymer is suitably used in a blood purifier, particularly a continuous blood purifier.

## EXAMPLES

The present invention will be described by way of Examples, but the present invention is not limited to these Examples.

In Examples and Comparative Examples, the following abbreviations are used.

PVP: Polyvinylpyrrolidone
PVAc: Polyvinyl acetate
PNVA/PtVA: N-vinylacetamide/vinyl pivalate random copolymer
PNIPAM/PEPR: N-isopropylacrylamide/ethyl acrylate random copolymer
PVP/PVAc: Vinylpyrrolidone/vinyl acetate random copolymer
PVP/PVPr: Vinylpyrrolidone/vinyl propionate random copolymer
PVP/PtVA: Vinylpyrrolidone/vinyl pivalate random copolymer
PVP/PVBu: Vinylpyrrolidone/vinyl butyrate random copolymer
PVP/PVBa: Vinylpyrrolidone/vinyl benzoate random copolymer
PVP/PVDe: Vinylpyrrolidone/vinyl decanoate random copolymer
PVP/PVNo: Vinylpyrrolidone/vinyl nonanoate random copolymer
PVP/PVP6: Vinylpyrrolidone /1-vinyl-2-piperidone random copolymer
ACMO/PVP: Acryloylmorpholine/vinylpyrrolidone random copolymer
PVCL/PS: Vinyl caprolactam/polystyrene random copolymer

### <Evaluation Method>

(1) Hydration energy density of copolymer

The hydration energy of the monomer unit obtained from quantum chemical calculation is defined by the molecular model of the monomer unit shown below.

With respect to the molecular model of the monomer unit, when the repeating unit is a structure shown by the following chemical formula (V), a structure shown by the following chemical formula (VI) was included in calculation. As an example, the case of vinyl propionate was described.

[Chemical Formula 4]

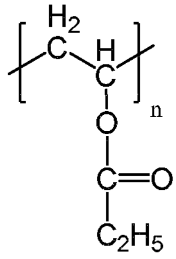

(V)

[Chemical Formula 5]

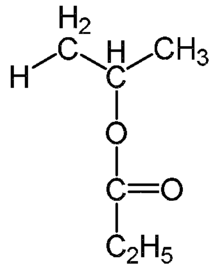

(VI)

Gaussian09, Revision D.01 (registered trademark) manufactured by Gaussian, Inc. was used for quantum chemical calculation, and MaterialsStudio (registered trademark) manufactured by BIOVIA Corp. was used for Connollysurface.

The hydration energy of the monomer unit was calculated by the following method.

First, the structure of the monomer unit in vacuum was optimized, and then energy in vacuum and energy in water were calculated for the optimized structure.

In the structure optimization step, density functional theory was used. B3LYP was used for the functional, and 6-31G(d,p) was used for the basis function. In addition, opt was set as a keyword entered in an input file.

The energy in vacuum was calculated using density functional theory. B3LYP was used for the functional, and 6-31G(d,p) was used for the basis function.

The energy in water was calculated using density functional theory. B3LYP was used for the functional, and 6-31G(d,p) was used for the basis function. In addition, a polarizable continuum model was used for calculation of energy in water, and the following parameters were used as keywords:

SCRF = (PCM, G03Defaults, Read, Solvent = Water)
Radii = UAHF
Alpha = 1.20

The volume of the monomer unit was calculated using the Connollysurface method. In that case, the parameters set were as follows: Gridresolution = Coarse

Gridinterval = 0.75Å (0.075 nm)
vdWfactor = 1.0
Connollyradius = 1.0Å (0.1 nm)

The hydration energy density of the copolymer is defined by the abovementioned Formula (1) based on the hydration energy and the volume calculated by the Connollysurface method. The volume of the monomer unit in the abovementioned Formula (1) was the optimized structure.

(2) Hydration energy density of monomer unit i

The hydration energy density of monomer unit i was calculated based on the abovementioned Formula (2).

(3) Volume fraction of monomer unit with highest hydration energy density of monomer unit i

The volume fraction of a monomer unit with a highest hydration energy density of monomer unit i was calculated based on the abovementioned Formula (3).

(4) Difference in hydration energy density

The difference in hydration energy density was calculated based on the abovementioned Formula (4).

The hydration energy density of the copolymer, the presence or absence of a hydroxy group, the volume fraction of a monomer unit with a highest hydration energy density of monomer unit i, and the difference in hydration energy density calculated in the following Examples and Comparative Examples are shown in Table 1. Examples 1-8 and 11-13 are reference examples.

[Table 1]

| | Name of polymer | Hydration energy density of copolymer ($kJ \cdot mol^{-1} \cdot nm^{-3}$) | Difference in hydration energy density ($kJ \cdot mol^{-1} \cdot nm^{-3}$) | Presence or absence of hydroxy group |
|---|---|---|---|---|
| Example 1 | PVP/PVPr (molar ratio 60:40) | 182.422 | 82.006 | C |
| Example 2 | PVP/PtVA (molar ratio 70:30) | 171.962 | 124.265 | C |
| Example 3 | PVP/PVPr (molar ratio 70:30) | 190.372 | 82.006 | C |
| Example 4 | PVP/PVBu (molar ratio 80:20) | 193.301 | 96.232 | C |
| Example 5 | PVP/PVBa (molar ratio 80:20) | 189.535 | 102.926 | C |
| Example 6 | PVP/PVDe (molar ratio 80:20) | 167.778 | 143.930 | C |
| Example 7 | PVP/PVNo (molar ratio 80:20) | 171.126 | 140.164 | C |
| Example 8 | PVP/PVPr (molar ratio 40:60) | 165.686 | 82.006 | C |

| | | | | |
|---|---|---|---|---|
| Example 9 | PNVA/PtVA (molar ratio 50:50) | 177.820 | 215.894 | C |
| Example 10 | PNIPAM/PEPR (molar ratio 50:50) | 180.749 | 91.211 | C |
| Comparative Example 1 | PVP | 213.384 | - | C |
| Comparative Example 2 | PVAc | 152.716 | - | C |
| Comparative Example 3 | PVP/PVAc (weight ratio 60:40) | 189.117 | 60.668 | C |
| Comparative Example 4 | PVP/PVP6 (molar ratio 60:40) | 194.974 | 42.677 | C |
| Comparative Example 5 | ACMO/PVP (molar ratio 60:40) | 222.589 | 131.378 | C |
| Comparative Example 6 | PVCL/PS (molar ratio 60:40) | 141.419 | 76.567 | C |
| Comparative Example 7 | PVP/PVPr (molar ratio 30:70) | 157.318 | 82.006 | C |

[Table 1 (continued)]

| | Volume fraction of monomer unit with highest hydration energy density of monomer unit i (%) | Comprehensive evaluation | Reduction rate of water permeability (%) | Amount of adhered platelets (number) | Reduction rate of sieving coefficient of albumin (%) |
|---|---|---|---|---|---|
| Example 1 | 62.2 | A | 7 | 0 | 2 |
| Example 2 | 66.6 | A | 9 | 0 | 3 |
| Example 3 | 71.9 | B | 13 | 0 | 7 |
| Example 4 | 79.4 | B | 8 | 0 | 9 |
| Example 5 | 76.9 | B | 5 | 4 | 8 |
| Example 6 | 68.4 | B | 3 | 2 | 17 |
| Example 7 | 69.8 | B | 10 | 1 | 25 |
| Example 8 | 42.3 | B | 12 | 2 | 8 |

| | | | | | |
|---|---|---|---|---|---|
| Example 9 | 41.2 | A | 6 | 0 | 2 |
| Example 10 | 56.0 | A | 7 | 0 | 2 |
| Comparative Example 1 | 100.0 | C | 55 | 21 | 60 |
| Comparative Example 2 | 100.0 | C | 35 | 21 | 29 |
| Comparative Example 3 | 59.9 | C | 32 | 2 | 15 |
| Comparative Example 4 | 57.5 | C | 28 | 18 | 26 |
| Comparative Example 5 | 63.9 | C | 34 | 40 | 45 |
| Comparative Example 6 | 66.5 | C | 28 | 46 | 39 |
| Comparative Example 7 | 32.0 | C | 27 | 4 | 10 |

In Table 1, as comprehensive evaluation, the case where the reduction rate of water permeability is 15% or less, the number of adhered platelets is 5 or less, and the reduction rate of the sieving coefficient of albumin is 25% or less was evaluated as B. Furthermore, the case where the reduction rate of water permeability is 10% or less, the number of adhered platelets is 0 or less, and the reduction rate of the sieving coefficient of albumin is 5% or less was evaluated as A. The other cases were evaluated as C. The case of a monomer unit containing a hydroxy group was evaluated as B, and the case of a monomer unit not containing a hydroxy group was evaluated as C.

(5) Number average molecular weight

A 0.1 N $LiNO_3$ solution of water/methanol = 50/50 (volume ratio) was prepared and used as a GPC developing solution. In 2 ml of the solution, 2 mg of a copolymer was dissolved. Into a Prominence GPC system manufactured by Shimadzu Corporation, 100 μL of this copolymer solution was injected, and measurement was performed. The configuration of the apparatus is as follows.
pump: LC-20AD, auto sampler: SIL-20AHT, column oven: CTO-20A, detector: RID-10A, column: manufactured by Tosoh Corporation; $GMPW_{XL}$ (inner diameter 7.8 mm × 30 cm, particle size 13 μm). The flow rate was 0.5 mL/min, and the measurement time was 30 minutes. The detection was performed with a differential refractive index detector RID-10A (manufactured by Shimadzu Corporation), and the number average molecular weight was calculated from the peak derived from the copolymer that appeared around the elution time of 15 minutes. The number average molecular weight was calculated by rounding off the number to the nearest hundred. A polyethylene oxide standard sample (0.1 kD to 1258 kD) manufactured by Agilent was used for preparing a calibration curve.

(6) Molar fraction of hydrophilic monomer unit

In 2 ml of chloroform-D, 99.7% (manufactured by Wako Pure Chemical Industries, Ltd.; containing 0.05 V/V% TMS), 2 mg of the copolymer was dissolved, and the solution was put in an NMR sample tube and subjected to NMR (manufactured by JEOL Ltd.; superconducting FTNMREX-270) measurement. The temperature was set to room temperature, and the integration time was 32 times. From this measurement result, using the area of the region surrounded by the peak derived from the proton (3H) bonded to the carbon atom adjacent to the nitrogen atom of vinylpyrrolidone observed between 2.7 to 4.3 ppm and the baseline: $3A_{PVP}$, and the area of the region surrounded by the peak derived from the proton (1H) bonded to the carbon at the α-position of vinyl carboxylate observed between 4.3 to 5.2 ppm and the baseline: $A_{VC}$, the value of $A_{PVP}/(A_{PVP}+A_{VC}) \times 100$ was calculated and regarded as the molar fraction of the vinylpyrrolidone unit. This method is an example of measuring the molar fraction in a copolymer vinylpyrrolidone and vinyl carboxylate. In the case of a copolymer made of a combination of other monomers, peaks derived from appropriate protons are selected for the determination of the molar fraction. The molar fraction was calculated by rounding off the number to the nearest ten.

(7) Measurement of reduction rate of water permeability before and after circulation of bovine blood

First, a circuit was connected to an inlet and an outlet on a B side (blood side) of the hollow fiber module, and washed with water at a rate of 200 mL/min for 5 minutes. Next, water (37°C) was flowed at a rate of 200 mL/min, the outflow from the B outlet was adjusted, and a filtration amount V per 1 minute of outflow to a D side and an average pressure P of the B side inlet and outlet were measured. By changing outflow from the B outlet, measurement was performed at three points, and the average value of the value calculated by the following formula was regarded as water permeability 0 minutes after start of circulation [UFRP-0].

$$\text{UFRP (mL/hr/mmHg/m}^2\text{)} = V \times 60/P/A$$

V: Filtration amount(mL/min), P: Pressure (mmHg), A: Membrane area (m2)

Next, 2 L of bovine whole blood was circulated. A hollow fiber membrane module (1) and a blood circuit were connected as shown in Fig. 1. Bovine blood supplemented with heparin was adjusted so that the hematocrit was 30% and the total protein concentration was 6 to 7 g/dl, and put in a circulation beaker (4). The circulation beaker (4) containing the bovine blood was kept at 37°C in a warm water bath (9) equipped with a heater (8). An inlet of a Bi circuit (5), an outlet of a Bo circuit (6), and an outlet of an F circuit (7) were placed in the circulation beaker (4) containing 2 L of the bovine blood adjusted as mentioned above, and a Bi pump (2) was started at a circulation flow rate of 100 ml/min. After 60 minutes, the circulation was stopped. Then, the circuit was connected to an inlet and an outlet on a B side (blood side) of the hollow fiber module, and washed with physiological saline at a rate of 200 mL/min for 10 minutes. Furthermore, the circuit was washed with water at a rate of 200 mL/min for 5 minutes, and then water permeability 60 minutes after start of circulation [UFRP-60] was calculated in the same manner as mentioned above.

The reduction rate of water permeability was calculated by the following formula.

$$\text{Reduction rate of water permeability (\%)} = ([\text{UFRP-0}] - [\text{UFRP - 60}])/[\text{UFRP - 0}] \times 100$$

(8) Method for human platelet adhesion test of hollow fiber membrane

A double-sided tape was attached to a circular plate 18 mmϕ in diameter made of polystyrene, and a hollow fiber membrane irradiated with γ-rays at 25 kGy was fixed thereto. The attached hollow fiber membrane was trimmed to a semi-cylindrical shape with a single-edged blade to expose the inner surface of the hollow fiber membrane. If there is any contaminant, scratch, crease or the like on the inner surface of the hollow fiber, platelets may adhere to that portion and hinder correct evaluation, and thus attention should be paid. The circular plate was attached to a cylindrically cut Falcon (registered trademark) tube (18 mmϕ in diameter, No. 2051) so that the face to which the hollow fiber membrane was attached was inside of the cylinder, and the gap was filled with Parafilm. The inside of this cylindrical tube was washed with physiological saline, and then the tube was filled with physiological saline. Human venous blood was collected, and heparin was added to the blood immediately after collection so that the concentration would be 50 U/ml. The physiological saline in the cylindrical tube was discharged, and then 1.0 ml of the blood was put in the cylindrical tube within 10 minutes after the blood collection and shaken at 37°C for 1 hour. Then, the hollow fiber membrane was washed with 10 ml of physiological saline, and blood components were fixed with 2.5% glutaraldehyde physiological saline and washed with 20 ml of distilled water. The washed hollow fiber membrane was dried under reduced pressure at 20°C and 0.5 Torr for 10 hours. This hollow fiber membrane was attached to a sample stage of a scanning electron microscope with a double-sided tape. After that, a Pt-Pd thin film was formed on the hollow fiber membrane surface by sputtering to prepare a sample. The inner surface of this hollow fiber membrane sample was observed with a field emission type scanning electron microscope (manufactured by Hitachi, Ltd.; S800) at a magnification of 1500 times, and the number of adhered platelets in one field of view ($4.3 \times 10^3\ \mu m^2$) was counted. When 50 or more platelets adhered, it was assumed that no platelet adhesion suppression effect was exerted, and the number of adhered platelets was regarded as 50. Since a pool of blood tends to occur at an end portion in the longer direction of the hollow fiber, the average value of the number of adhered platelets in 20 different fields of view near the center of the hollow fiber membrane was regarded as the number of adhered platelets (number/$4.3 \times 10^3\ \mu m^2$).

(9) Reduction rate of sieving coefficient of albumin

The sieving coefficient of albumin was measured as follows. First, a hollow fiber membrane module (1) and a

blood circuit were connected as shown in Fig. 1. Bovine blood supplemented with heparin was adjusted so that the hematocrit was 30% and the total protein concentration was 6 to 7 g/dl, and put in a circulation beaker (4). The circulation beaker (4) containing the bovine blood was kept at 37°C in a warm water bath (9) equipped with a heater (8).

An inlet of a Bi circuit (5), an outlet of a Bo circuit (6), and an outlet of an F circuit (7) were placed in the circulation beaker (4) containing 2 L of the bovine blood adjusted as mentioned above, and a Bi pump (2) was started at a circulation flow rate of 100 ml/min.

Subsequently, the F pump (3) was started at a filtration flow rate of 10 ml/min, and the blood was sampled over time at the inlet of the Bi circuit (5), the outlet of the Bo circuit (6), and the outlet of the F circuit (7).

The albumin concentration at each elapsed time from the start of the F pump (3) was measured, and the sieving coefficient of albumin (ScAlb) at each elapsed time was calculated according to the following formula.

$$\text{ScAlb (\%)} = \text{CF}/\{0.5 \times (\text{CBi} + \text{CBo})\} \times 100$$

wherein, CF represents the albumin concentration (g/ml) at the outlet of the F circuit (7), CBo represents the albumin concentration (g/ml) at the outlet of the Bo circuit (6), and CBi represents the albumin concentration (g/ml) at the inlet of the Bi circuit (5).

The reduction rate of the sieving coefficient of albumin after a perfusion time of 60 minutes (ScAlb60) to the sieving coefficient of albumin after a perfusion time of 10 minutes (ScAlb10) was calculated according to the following formula. The reduction rate was calculated by rounding off the number to the nearest whole number.

$$\text{Reduction rate (\%)} = (\text{ScAlb10} - \text{ScAlb60})/\text{ScAlb10} \times 100$$

(10) Method for thrombus formation test on PET filter

A PET filter was cut into a 1 cm × 1 cm piece, and placed in a cylindrical container made of polypropylene with a diameter of 1 cm and a depth of 0.8 cm. To this, 1 ml of human blood supplemented with heparin so that the concentration became 50 U/ml was added so that the filter was immersed, and then shaken for 30 minutes. The filter was taken out, and whether a thrombus was formed or not was confirmed.

<Method for manufacturing hollow fiber membrane module>

To 72 parts by weight of N,N-dimethylacetamide and 1 part by weight of water, 18 parts by weight of polysulfone (manufactured by Teijin Amoco; Udel P-3500) and 9 parts by weight of polyvinylpyrrolidone (manufactured by BASF Corporation; K30) were added, and the mixture was heated at 90°C for 14 hours for dissolution. This membrane-forming stock solution was discharged from an orifice-type double cylindrical spinneret having an outer diameter of 0.3 mm and an inner diameter of 0.2 mm, and an solution of 57.5 parts by weight of N,N-dimethylacetamide and 42.5 parts by weight of water was discharged as a core liquid, the membrane-forming stock solution and the core liquid were passed through a dry part having a length of 350 mm, and led to a coagulation bath of 100% water to obtain a hollow fiber. The hollow fiber thus obtained had an inner diameter of 200 μm and a membrane thickness of 40 μm. Through a plastic tube, 50 hollow fibers were passed, and a plastic tube minimodule having an effective length of 100 mm whose both ends were fixed by an adhesive was prepared. An aqueous solution in which the polymer was dissolved was passed from the blood side inlet to the dialysate side inlet of the minimodule. Furthermore, a 0.1% by weight aqueous ethanol solution was passed from the blood side inlet to the dialysate side inlet of the hollow fiber membrane module and from the blood side inlet to the blood side outlet thereof, and the module was irradiated with 25 kGy γ-rays to prepare a hollow fiber membrane module.

<Method for manufacturing PET filter>

A polyethylene terephthalate filter (manufactured by Toray Industries, Inc.) having a membrane thickness of 5 μm was cut into a 5cm$^2$ piece and placed in a 15 mL centrifuge tube (manufactured by AS ONE Corporation). The interior of the centrifuge tube was filled with an aqueous copolymer solution having a concentration of 0.1 ppm, the tube was covered, and the filter was irradiated with 25 kGy γ-rays to obtain a PET filter.

(Example 1) (Reference example)

A vinylpyrrolidone/vinyl propionate random copolymer was prepared by the following method. That is, 19.5 g

of a vinylpyrrolidone monomer, 17.5 g of a vinyl propionate monomer, 56 g of t-amyl alcohol as a polymerization solvent, and 0.175 g of 2,2'-azobis(2,4-dimethylvaleronitrile) as a polymerization initiator were mixed, and the mixture was stirred at 70°C for 6 hours in a nitrogen atmosphere. The reaction liquid was cooled to room temperature to stop the reaction, concentrated, and then charged into hexane. The deposited white precipitate was collected and dried under reduced pressure to obtain 21.0 g of a copolymer. From the result of $^1$H-NMR, it was found that the molar fraction of the vinylpyrrolidone monomer unit was 60%. Furthermore, from the measurement result of GPC, the number average molecular weight was 16,500.

A separation membrane module for medical use having a shape of a hollow fiber membrane in which the prepared vinylpyrrolidone/vinyl propionate random copolymer was introduced onto the surface of the polysulfone hollow fiber was prepared by the following method. A 1.0% by weight aqueous ethanol solution in which 300 ppm of the copolymer was dissolved was passed from the blood side inlet to the dialysate side inlet of the hollow fiber membrane module prepared by the method for manufacturing a hollow fiber membrane module. Furthermore, a 0.1% by weight aqueous ethanol solution was passed from the blood side inlet to the dialysate side inlet of the hollow fiber membrane module and from the blood side inlet to the blood side outlet thereof, and the module was irradiated with 25 kGy $\gamma$-rays to prepare a separation membrane module for medical use. From the measurement result of ATR-IR, it was found that the introduction amount (area ratio) of the copolymer on the inner surface of the hollow fiber was 0.06 on average. The reduction rate of water permeability of the prepared separation membrane module for medical use, the amount of adhered platelets of the hollow fiber membrane, and the sieving coefficient of albumin of the separation membrane module for medical use were measured. As a result, as shown in Table 1, the reduction rate of water permeability was 7%, the amount of adhered platelets was 0, and the reduction rate of the sieving coefficient of albumin after a perfusion time of 60 minutes to the sieving coefficient of albumin after a perfusion time of 10 minutes was 2%.

(Example 2) (Reference example)

A separation membrane module for medical use was prepared in the same manner as in Example 1 except that a vinylpyrrolidone/vinyl pivalate random copolymer (molar fraction of vinylpyrrolidone monomer unit: 70%, number average molecular weight: 3,900) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 9%, the amount of adhered platelets was 0, and the reduction rate of the sieving coefficient of albumin after a perfusion time of 60 minutes to the sieving coefficient of albumin after a perfusion time of 10 minutes was 3%.

(Example 3) (Reference example)

A separation membrane module for medical use was prepared in the same manner as in Example 1 except that a vinylpyrrolidone/vinyl propionate random copolymer (molar fraction of vinylpyrrolidone monomer unit: 70%, number average molecular weight: 20,800) was used, and the reduction rate of water permeability, the amount of adhered platelets, and the sieving coefficient of albumin were measured. As a result, as shown in Table 1, the reduction rate of water permeability was 13%, the amount of adhered platelets was 0, and the reduction rate of the sieving coefficient of albumin after a perfusion time of 60 minutes to the sieving coefficient of albumin after a perfusion time of 10 minutes was 7%.

(Example 4) (Reference example)

A separation membrane module for medical use was prepared in the same manner as in Example 1 except that a vinylpyrrolidone/vinyl butyrate random copolymer (molar fraction of vinylpyrrolidone monomer unit: 80%, number average molecular weight: 2,100) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 8%, the amount of adhered platelets was 0, and the reduction rate of the sieving coefficient of albumin after a perfusion time of 60 minutes to the sieving coefficient of albumin after a perfusion time of 10 minutes was 9%.

(Example 5) (Reference example)

A separation membrane module for medical use was prepared in the same manner as in Example 1 except that a copolymer as a vinylpyrrolidone/vinyl benzoate random copolymer (molar fraction of vinylpyrrolidone monomer unit: 80%, number average molecular weight: 2,900) was used in place of the vinylpyrrolidone/vinyl propionate random copolymer, and the reduction rate of water permeability, the amount of adhered platelets, and the sieving coefficient of

albumin were measured. As a result, as shown in Table 1, the reduction rate of water permeability was 5%, the amount of adhered platelets was 4, and the reduction rate of the sieving coefficient of albumin after a perfusion time of 60 minutes to the sieving coefficient of albumin after a perfusion time of 10 minutes was 8%.

(Example 6) (Reference example)

A separation membrane module for medical use was prepared in the same manner as in Example 1, except that a vinylpyrrolidone/vinyl decanoate random copolymer (molar fraction of vinylpyrrolidone monomer unit: 80%, number average molecular weight: 19,000) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 3%, the amount of adhered platelets was 2, and the reduction rate of the sieving coefficient of albumin after a perfusion time of 60 minutes to the sieving coefficient of albumin after a perfusion time of 10 minutes was 17%.

(Example 7) (Reference example)

A separation membrane module for medical use was prepared in the same manner as in Example 1, except that a vinylpyrrolidone/vinyl nonanoate random copolymer (molar fraction of vinylpyrrolidone monomer unit: 80%, number average molecular weight: 4,400) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 10%, the amount of adhered platelets was 1, and the reduction rate of the sieving coefficient of albumin after a perfusion time of 60 minutes to the sieving coefficient of albumin after a perfusion time of 10 minutes was 25%.

(Example 8) (Reference example)

A separation membrane module for medical use was prepared in the same manner as in Example 1, except that, among vinylpyrrolidone/vinyl propionate random copolymers, one having a molar fraction of vinylpyrrolidone of 40% and a number average molecular weight of 20,800 was used, and the reduction rate of water permeability, the amount of adhered platelets, and the sieving coefficient of albumin were measured. As a result, as shown in Table 1, the reduction rate of water permeability was 12%, the amount of adhered platelets was 2, and the reduction rate of the sieving coefficient of albumin after a perfusion time of 60 minutes to the sieving coefficient of albumin after a perfusion time of 10 minutes was 8%.

(Example 9)

A separation membrane module for medical use having a shape of a hollow fiber membrane in which an N-vinylacetamide/vinyl pivalate random copolymer (molar fraction of N-vinylacetamide unit: 50%, number average molecular weight: 7,700) was introduced onto the surface of the polysulfone hollow fiber was prepared by the following method. A 10% by weight aqueous ethanol solution in which 100 ppm of the copolymer was dissolved was passed from the blood side inlet to the dialysate side inlet of the hollow fiber membrane module prepared by the method for manufacturing a hollow fiber membrane module. Furthermore, a 0.1% by weight aqueous ethanol solution was passed from the blood side inlet to the dialysate side inlet of the hollow fiber membrane module and from the blood side inlet to the blood side outlet thereof, and the module was irradiated with 25 kGy $\gamma$-rays to prepare a separation membrane module for medical use. From the measurement result of ATR-IR, it was found that the introduction amount (area ratio) of the copolymer on the inner surface of the hollow fiber was 0.06 on average. The reduction rate of water permeability of the prepared separation membrane module for medical use, the amount of adhered platelets of the hollow fiber membrane, and the sieving coefficient of albumin of the separation membrane module for medical use were measured. As a result, as shown in Table 1, the reduction rate of water permeability was 6%, the amount of adhered platelets was 0, and the reduction rate of the sieving coefficient of albumin after a perfusion time of 60 minutes to the sieving coefficient of albumin after a perfusion time of 10 minutes was 2%.

(Example 10)

A separation membrane module for medical use having a shape of a hollow fiber membrane in which an N-isopropylacrylamide/ethyl acrylate random copolymer (molar fraction of N-isopropylacrylamide unit: 50%, number average molecular weight: 3,000) was introduced onto the surface of the polysulfone hollow fiber was prepared by the following method. A 1.0% by weight aqueous ethanol solution in which 100 ppm of the copolymer was dissolved was

passed from the blood side inlet to the dialysate side inlet of the hollow fiber membrane module prepared by the method for manufacturing a hollow fiber membrane module. Furthermore, a 0.1% by weight aqueous ethanol solution was passed from the blood side inlet to the dialysate side inlet of the hollow fiber membrane module and from the blood side inlet to the blood side outlet thereof, and the module was irradiated with 25 kGy γ-rays to prepare a separation membrane module for medical use. From the measurement result of ATR-IR, it was found that the introduction amount (area ratio) of the copolymer on the inner surface of the hollow fiber was 0.05 on average. The reduction rate of water permeability of the prepared separation membrane module for medical use, the amount of adhered platelets of the hollow fiber membrane, and the sieving coefficient of albumin of the separation membrane module for medical use were measured. As a result, as shown in Table 1, the reduction rate of water permeability was 7%, the amount of adhered platelets was 0, and the reduction rate of the sieving coefficient of albumin after a perfusion time of 60 minutes to the sieving coefficient of albumin after a perfusion time of 10 minutes was 2%.

(Comparative Example 1)

A separation membrane module for medical use was prepared in the same manner as in Example 1, except that polyvinylpyrrolidone (manufactured by BASF Corporation; K90) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 55%, the amount of adhered platelets was 21, and the reduction rate of the sieving coefficient of albumin after a perfusion time of 60 minutes to the sieving coefficient of albumin after a perfusion time of 10 minutes was 60%.

(Comparative Example 2)

A separation membrane module for medical use was prepared in the same manner as in Example 1, except that polyvinyl acetate (manufactured by BASF Corporation; K90) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 35%, the amount of adhered platelets was 21, and the reduction rate of the sieving coefficient of albumin after a perfusion time of 60 minutes to the sieving coefficient of albumin after a perfusion time of 10 minutes was 29%.

(Comparative Example 3)

A separation membrane module for medical use was prepared in the same manner as in Example 1, except that a vinylpyrrolidone/vinyl acetate random copolymer (manufactured by BASF Corporation; Kollidon VA64) was used in place of the vinylpyrrolidone/vinyl propionate random copolymer, and the sieving coefficient of albumin was measured. As a result, as shown in Table 1, the reduction rate of water permeability was 32%, the amount of adhered platelets was 2, and the reduction rate of the sieving coefficient of albumin after a perfusion time of 60 minutes to the sieving coefficient of albumin after a perfusion time of 10 minutes was 15%.

(Comparative Example 4)

A separation membrane module for medical use was prepared in the same manner as in Example 1, except that a vinylpyrrolidone/1-vinyl-2-piperidone copolymer (molar fraction of vinylpyrrolidone monomer unit: 60%, number average molecular weight: 5,100) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 28%, the amount of adhered platelets was 18, and the reduction rate of the sieving coefficient of albumin after a perfusion time of 60 minutes to the sieving coefficient of albumin after a perfusion time of 10 minutes was 26%.

(Comparative Example 5)

A separation membrane module for medical use was prepared in the same manner as in Example 1, except that an acryloylmorpholine/vinylpyrrolidone random copolymer (molar fraction of acryloylmorpholine: 60%, number average molecular weight: 6,200) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 34%, the amount of adhered platelets was 40, and the reduction rate of the sieving coefficient of albumin after a perfusion time of 60 minutes to the sieving coefficient of albumin after a perfusion time of 10 minutes was 45%.

(Comparative Example 6)

A separation membrane module for medical use was prepared in the same manner as in Example 1, except that a vinyl caprolactam/polystyrene random copolymer (molar fraction of vinyl caprolactam monomer unit: 60%, number average molecular weight: 7,300) was used in place of the vinylpyrrolidone/vinyl propionate random copolymer, and the reduction rate of water permeability, the amount of adhered platelets, and the sieving coefficient of albumin were measured. As a result, as shown in Table 1, the reduction rate of water permeability was 28%, the amount of adhered platelets was 46, and the reduction rate of the sieving coefficient of albumin after a perfusion time of 60 minutes to the sieving coefficient of albumin after a perfusion time of 10 minutes was 39%.

(Comparative Example 7)

A separation membrane module for medical use was prepared in the same manner as in Example 1, except that, among vinylpyrrolidone/vinyl propionate random copolymers, one having a molar fraction of vinylpyrrolidone of 30% and a number average molecular weight of 10,800 was used, and the reduction rate of water permeability, the amount of adhered platelets, and the sieving coefficient of albumin were measured. As a result, as shown in Table 1, the reduction rate of water permeability was 27%, the amount of adhered platelets was 4, and the reduction rate of the sieving coefficient of albumin after a perfusion time of 60 minutes to the sieving coefficient of albumin after a perfusion time of 10 minutes was 10%.

(Example 11) (Reference example)

Using a vinylpyrrolidone/vinyl propionate random copolymer (molar fraction of vinylpyrrolidone monomer unit: 60%, number average molecular weight: 16,500) as the copolymer, a PET filter was prepared by the method for manufacturing a PET filter. A thrombus formation test of the PET filter thus obtained showed that no thrombus was formed as shown in Table 2.

(Example 12) (Reference example)

Using a vinylpyrrolidone/vinyl pivalate random copolymer (molar fraction of vinylpyrrolidone monomer unit: 70%, number average molecular weight: 3,900) as the copolymer, a PET filter was prepared by the method for manufacturing a PET filter. A thrombus formation test of the PET filter thus obtained showed that no thrombus was formed as shown in Table 2.

(Example 13) (Reference example)

Using a vinylpyrrolidone/vinyl butyrate random copolymer (molar fraction of vinylpyrrolidone monomer unit: 60%, number average molecular weight: 8,500) as the copolymer, a PET filter was prepared by the method for manufacturing a PET filter. A thrombus formation test of the PET filter thus obtained showed that no thrombus was formed as shown in Table 2.

(Example 14)

Using an N-vinylacetamide/vinyl pivalate random copolymer (molar fraction of vinylpyrrolidone monomer unit: 50%, number average molecular weight: 7,700) as the copolymer, a PET filter was prepared by the method for manufacturing a PET filter. A thrombus formation test of the PET filter thus obtained showed that no thrombus was formed as shown in Table 2.

(Example 15)

Using an N-isopropylacrylamide/ethyl acrylate random copolymer (molar fraction of N-isopropylacrylamide monomer unit: 50%, number average molecular weight: 3,000) as the copolymer, a PET filter was prepared by the method for manufacturing a PET filter. A thrombus formation test of the PET filter thus obtained showed that no thrombus was formed as shown in Table 2.

(Comparative Example 8)

A PET filter was prepared in the same manner as in Example 11, except that no copolymer was used and a

thrombus formation test was performed. As a result, a thrombus was formed as shown in Table 2.

(Comparative Example 9)

A PET filter was prepared in the same manner as in Example 11, except that polyvinylpyrrolidone (manufactured by BASF Corporation; K30) was used in place of the vinylpyrrolidone/vinyl propionate random copolymer and a platelet adhesion test was performed. As a result, a thrombus was formed as shown in Table 2.

[Table 2]

| | Name of polymer | Situation of thrombus formation |
|---|---|---|
| Example 11 | PVP/PVPr (molar ratio 60:40) | Absent |
| Example 12 | PVP/PtVA (molar ratio 70:30) | Absent |
| Example 13 | PVP/PVBu (molar ratio 80:20) | Absent |
| Example 14 | PNVA/PtVA (molar ratio 50:50) | Absent |
| Example 15 | PNIPAM/PEPR (molar ratio 50:50) | Absent |
| Comparative Example 8 | None | Present |
| Comparative Example 9 | PVP | Present |

INDUSTRIAL APPLICABILITY

The copolymer of the present invention has an effect to suppress adhesion of proteins and platelets, and therefore can be used as a separation membrane and a medical device using the separation membrane. Particularly, the copolymer can be used as a blood purifier.

Description of Reference Symbols

1 Hollow fiber membrane module

2 Bi pump

3 F pump

4 Circulation beaker

5 Bi circuit

6 Bo circuit

7 F circuit

8 Heater

9 Warm water bath

**Claims**

**1.** A copolymer, comprising two or more types of monomer units, wherein

the two or more types of monomer units comprise a hydrophobic monomer unit and a hydrophilic monomer unit, the hydrophobic monomer unit is a monomer unit with a lower hydration energy density than that of a hydrophilic

monomer unit and is a repeating unit in a copolymer obtained by polymerizing monomers selected from the group consisting of vinyl carboxylate, acrylate, and a styrene derivative,

the hydrophilic monomer unit is a monomer unit with a higher hydration energy density than that of a hydrophobic monomer unit and is a repeating unit in a copolymer obtained by polymerizing monomers selected from the group consisting of an allylamine derivative, a vinylamine derivative, N-vinylamide, an acrylamide derivative, a methacrylamide derivative, and N-acryloylmorpholine, and

wherein

a hydration energy density of the copolymer calculated based on the following Formula (1) is 167.360 to 209.200 $kJ \cdot mol^{-1} \cdot nm^{-3}$,

the monomer unit with the highest hydration energy density of monomer unit i calculated based on the following Formula (2) is a monomer unit not containing a hydroxy group,

a volume fraction of the monomer unit with the highest hydration energy density of monomer unit i calculated based on the following Formula (3) is 35 to 90%,

a difference in hydration energy density calculated by the following Formula (4) is 71.128 to 418.400 $kJ \cdot mol^{-1} \cdot nm^{-3}$,

$$\text{Hydration energy density } (kJ \cdot mol^{-1} \cdot nm^{-3}) \text{ of copolymer} = \sum_{i=1}^{N} \{(\text{molar fraction of monomer unit i}) \times (\text{hydration energy of monomer unit i})\} / \sum_{i=1}^{N} \{(\text{molar fraction of monomer unit i}) \times (\text{volume of monomer unit i})\} \quad \bullet\bullet\bullet \text{ Formula (1)},$$

wherein the hydration energy of monomer unit i is an absolute value of a value obtained by subtracting energy in vacuum of monomer unit i from energy in water of monomer unit i, N represents a total number of monomer species constituting the copolymer, and i represents an integer of 1 or more and N or less,

$$\text{Hydration energy density } (kJ \cdot mol^{-1} \cdot nm^{-3}) \text{ of monomer unit i} = (\text{hydration energy of monomer unit i}) / (\text{volume of monomer unit i}) \quad \bullet\bullet\bullet \text{ Formula (2)},$$

$$\text{Volume fraction (\%) of monomer unit with highest hydration energy density of monomer unit i} = \text{molar fraction of monomer unit with highest hydration energy density of monomer unit i} \times \text{volume of monomer unit with highest hydration energy density of monomer unit i} / \sum_{i=1}^{N} \{(\text{molar fraction of monomer unit i}) \times (\text{volume of monomer unit i})\} \quad \bullet\bullet\bullet \text{ Formula (3)},$$

wherein N and i are the same as defined above, and

$$\text{Difference in hydration energy density } (kJ \cdot mol^{-1} \cdot nm^{-3}) = (\text{hydration energy density of monomer unit with highest hydration energy density of monomer unit i}) - (\text{hydration energy density of monomer unit with lowest hydration energy density of monomer unit i}) \quad \bullet\bullet\bullet \text{ Formula (4)}.$$

2. The copolymer according to claim 1, wherein

the hydration energy density of the copolymer is 167.360 to 200.832 $kJ \cdot mol^{-1} \cdot nm^{-3}$,

the volume fraction of the monomer unit with the highest hydration energy density of monomer unit i is 40 to 80%, and

the difference in hydration energy density is 71.128 to 313.800 $kJ \cdot mol^{-1} \cdot nm^{-3}$.

3. The copolymer according to any of claims 1 to 2, wherein the hydrophobic monomer unit is a monomer unit with a lower hydration energy density than that of a hydrophilic monomer unit and a repeating unit in a copolymer obtained by polymerizing monomers of vinyl carboxylate.

**4.** A separation membrane, comprising the copolymer according to any one of claims 1 to 3.

**5.** A medical device, comprising the copolymer according to any one of claims 1 to 3.

**6.** A blood purifier, comprising the separation membrane according to claim 4.

**Patentansprüche**

**1.** Copolymer, das zwei oder mehr Typen von Monomereinheiten umfasst, wobei:

die zwei oder mehr Typen von Monomereinheiten eine hydrophobe Monomereinheit und eine hydrophile Monomereinheit umfassen,
die hydrophobe Monomereinheit eine Monomereinheit mit einer niedrigeren Hydrierungsenergiedichte als derjenigen einer hydrophilen Monomereinheit ist und eine sich wiederholende Einheit in einem Copolymer ist, das durch das Polymerisieren von Monomeren erhalten wird, die aus der Gruppe ausgewählt werden, die aus Vinylcarboxylat, Acrylat und einem Styren-Derivat besteht,
die hydrophile Monomereinheit eine Monomereinheit mit einer höheren Hydrierungsenergiedichte als derjenigen einer hydrophoben Monomereinheit ist und eine sich wiederholende Einheit in einem Copolymer ist, das durch das Polymerisieren von Monomeren erhalten wird, die aus der Gruppe ausgewählt werden, die aus einem Allylamin-Derivat, einem Vinylamin-Derivat, N-Vinylamid, einem Acrylamid-Derivat, einem Methacrylamid-Derivat und N-Acryloylmorpholin besteht,
und wobei:

die Hydrierungsenergiedichte des Copolymers, berechnet basierend auf der folgenden Formel (1), 167,360 bis 209,200 $kJ \cdot mol^{-1} \cdot nm^{-3}$ beträgt,
die Monomereinheit mit der höchsten Hydrierungsenergiedichte der Monomereinheit i, berechnet basierend auf der folgenden Formel (2), eine Monomereinheit, die keine Hydroxygruppe enthält, ist,
der Volumenanteil der Monomereinheit mit der höchsten Hydrierungsenergiedichte der Monomereinheit i, berechnet basierend auf der folgenden Formel (3), 35 bis 90% beträgt,
die Differenz der Hydrierungsenergiedichte, berechnet durch die folgende Formel (4) 71,128 bis 418,400 $kJ \cdot mol^{-1} \cdot nm^{-3}$ beträgt,

$$\text{Hydrierungsenergiedichte } (kJ \cdot mol^{-1} \cdot nm^{-3}) \text{ von Copolymer} = \sum_{i=1}^{N}\{\text{Stoffmengenanteil der Monomereinheit i}) \times (\text{Hydrierungsenergie der Monomereinheit i})\} / \sum_{i=1}^{N}\{\text{Stoffmengenanteil der Monomereinheit i}) \times (\text{Volumen der Monomereinheit i})\}$$

(Formel 1)

wobei die Hydrierungsenergie der Monomereinheit i ein absoluter Wert eines Werts ist, der durch das Subtrahieren der Energie in Vakuum der Monomereinheit i von der Energie in Wasser der Monomereinheit i erhalten wird, N eine Gesamtanzahl von das Copolymer bildenden Monomerspezies wiedergibt und i eine Ganzzahl von 1 oder größer und N oder kleiner wiedergibt,

$$\text{Hydrierungsenergiedichte } (kJ \cdot mol^{-1} \cdot nm^{-3}) \text{ der Monomereinheit i} = (\text{Hydrierungsenergie der Monomereinheit i}) / (\text{Volumen der Monomereinheit i})$$

(Formel 2)

$$\text{Volumenanteil (\%) der Monomereinheit mit der höchsten Hydrierungsenergiedichte der Monomereinheit i} = \text{Stoffmengenanteil der Monomereinheit mit der höchsten Hydrierungsenergiedichte der Monomereinheit} \times \text{Volumen der Monomereinheit mit der höchsten Hydrierungsenergiedichte der Monomereinheit i} / \sum_{i=1}^{N} \{\text{Stoffmengenanteil der Monomereinheit i}) \times (\text{Volumen der Monomereinheit i})\} \quad \text{(Formel 3)}$$

wobei N und i wie weiter oben angegeben definiert sind, und

$$\text{Differenz der Hydrierungsenergiedichte } (kJ \bullet mol^{-1} \bullet nm^{-3}) = (\text{Hydrierungsenergiedichte der Monomereinheit mit der höchsten Hydrierungsenergiedichte der Monomereinheit i}) - (\text{Hydrierungsenergiedichte der Monomereinheit mit der niedrigsten Hydrierungsenergiedichte der Monomereinheit i}) \quad \text{(Formel 4).}$$

**2.** Copolymer nach Anspruch 1, wobei:

die Hydrierungsenergiedichte des Copolymers 167,360 bis 200,832 $kJ \bullet mol^{-1} \bullet nm^{-3}$ beträgt,
der Volumenanteil der Monomereinheit mit der höchsten Hydrierungsenergiedichte der Monomereinheit i 40 bis 80% beträgt, und
die Differenz der Hydrierungsenergiedichte 71,128 bis 313,800 $kJ \bullet mol^{-1} \bullet nm^{-3}$ beträgt.

**3.** Copolymer nach Anspruch 1 oder 2, wobei die hydrophobe Monomereinheit eine Monomereinheit mit einer niedrigeren Hydrierungsenergiedichte als derjenigen einer hydrophilen Monomereinheit und eine sich wiederholende Einheit in einem Copolymer, das durch das Polymerisieren von Monomeren von Vinylcarboxylat erhalten wird, ist.

**4.** Trennmembran, die das Copolymer gemäß den Ansprüchen 1 bis 3 umfasst.

**5.** Medizinische Vorrichtung, die das Copolymer gemäß den Ansprüchen 1 bis 3 umfasst.

**6.** Blutreiniger, der die Trennmembran gemäß Anspruch 4 umfasst.

## Revendications

**1.** Copolymère comprenant deux types ou plus d'unités monomères, dans lequel

les deux types ou plus d'unités monomères comprennent une unité monomère hydrophobe et une unité monomère hydrophile,
l'unité monomère hydrophobe est une unité monomère ayant une densité d'énergie d'hydratation inférieure à celle d'une unité monomère hydrophile et est une unité de répétition dans un copolymère obtenu par polymérisation de monomères choisis dans le groupe constitué du carboxylate de vinyle, de l'acrylate et d'un dérivé du styrène,
l'unité monomère hydrophile est une unité monomère ayant une densité d'énergie d'hydratation supérieure à

celle d'une unité monomère hydrophobe et est une unité de répétition dans un copolymère obtenu par polymérisation de monomères choisis dans le groupe constitué d'un dérivé d'allylamine, d'un dérivé de vinylamine, de N-vinylamide, d'un dérivé d'acrylamide, d'un dérivé de méthacrylamide et de N-acryloylmorpholine,
dans lequel
une densité d'énergie d'hydratation du copolymère calculée sur la base de la formule (1) suivante est de 167,360 à 209,200 $kJ \cdot mol^{-1} \cdot nm^{-3}$,
l'unité monomère ayant la densité d'énergie d'hydratation la plus élevée de l'unité monomère i, calculée sur la base de la formule (2) suivante, est une unité monomère ne contenant pas de groupe hydroxy,
une fraction volumique de l'unité monomère ayant la densité d'énergie d'hydratation la plus élevée de l'unité monomère i, calculée sur la base de la formule (3) suivante, est de 35 à 90 %,
une différence de densité d'énergie d'hydratation calculée par la formule (4) suivante est de 71,128 à 418,400 $kJ \cdot mol^{-1} \cdot nm^{-3}$, Densité d'énergie d'hydratation ($kJ \cdot mol^{-1} \cdot nm^{-3}$) du copolymère =

$\sum_{i=1}^{N}$ {(fraction molaire de l'unité monomère i) x (énergie d'hydratation de l'unité monomère i)}/

$\sum_{i=1}^{N}$ {(fraction molaire de l'unité monomère i) x (volume de l'unité monomère i)}

• • • Formule (1),

dans lequel l'énergie d'hydratation de l'unité monomère i est une valeur absolue d'une valeur obtenue par soustraction de l'énergie dans le vide de l'unité monomère i de l'énergie dans l'eau de l'unité monomère i, N représente un nombre total d'espèces monomères constituant le copolymère, et i représente un nombre entier de 1 ou plus et de N ou moins,

Densité d'énergie d'hydratation ($kJ \cdot mol^{-1} \cdot nm^{-3}$) de l'unité monomère i =
(énergie d'hydratation de l'unité monomère i)/(volume de l'unité monomère i) • • • Formule (2),

fraction volumique (%) de l'unité monomère ayant la densité d'énergie d'hydratation la plus élevée de l'unité monomère i = fraction molaire de l'unité monomère ayant la densité d'énergie d'hydratation la plus élevée de l'unité monomère i x
volume de l'unité monomère ayant la densité d'énergie d'hydratation la plus élevée de l'unité monomère i/

$\sum_{i=1}^{N}$ {(fraction molaire de l'unité monomère i) x (volume de l'unité monomère i)} • • • Formule (3),

N et i étant identiques à ceux définis ci-dessus, et

Différence de densité d'énergie d'hydratation ($kJ \cdot mol^{-1} \cdot nm^{-3}$) = (densité d'énergie d'hydratation de l'unité monomère ayant la densité d'énergie d'hydratation la plus élevée de l'unité monomère i) - (densité d'énergie d'hydratation de l'unité monomère ayant la densité d'énergie d'hydratation la plus faible de l'unité monomère i) • • • Formule (4).

**2.** Copolymère selon la revendication 1, dans lequel

la densité d'énergie d'hydratation du copolymère est de 167,360 à 200,832 $kJ \cdot mol^{-1} \cdot nm^{-3}$,
la fraction volumique de l'unité monomère ayant la densité d'énergie d'hydratation la plus élevée de l'unité monomère i est de 40 à 80 %, et
la différence de densité d'énergie d'hydratation est de 71,128 à 313,800 $kJ \cdot mol^{-1} \cdot nm^{-3}$.

**3.** Copolymère selon l'une quelconque des revendications 1 à 2, dans lequel l'unité monomère hydrophobe est une unité monomère ayant une densité d'énergie d'hydratation inférieure à celle d'une unité monomère hydrophile et d'une unité de répétition dans un copolymère obtenu par polymérisation de monomères de carboxylate de vinyle.

**4.** Membrane de séparation comprenant le copolymère selon l'une quelconque des revendications 1 à 3.

**5.** Dispositif médical comprenant le copolymère selon l'une quelconque des revendications 1 à 3.

**6.** Purificateur de sang, comprenant la membrane de séparation selon la revendication 4.

[Fig. 1]

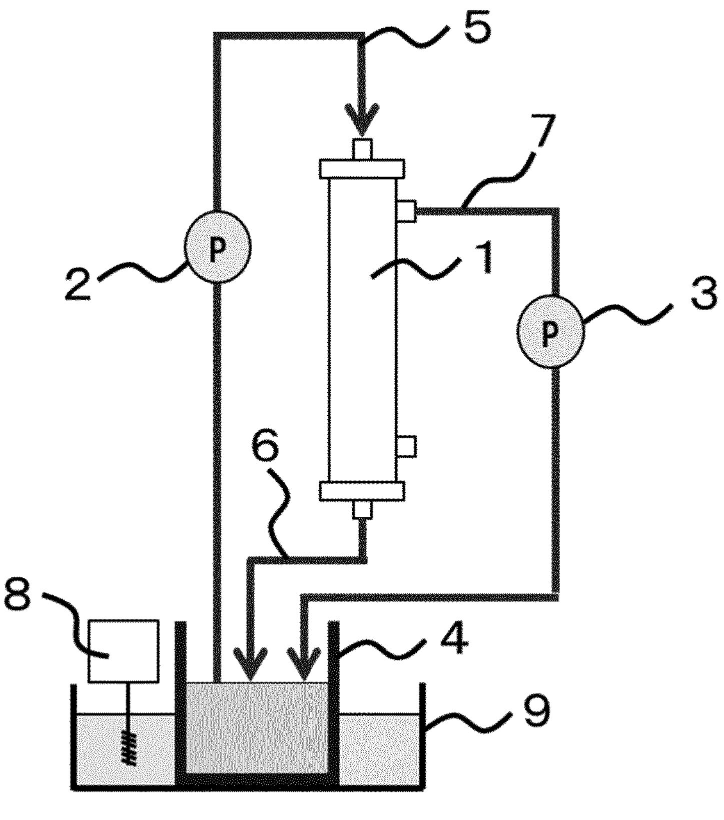

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H218695 B
- JP H8131791 A
- JP H6238139 A
- JP 2011072987 A


### Non-patent literature cited in the description


- **KAZUNORI KATAOKA et al.** Nano Bioengineering. Kyorin Tosho, October 2007, 115-116
- **M. TIJINK et al.** *Journal of materials chemistry B,* 10 July 2013, vol. 1 (44), 6066-6076
- **HIDEMUNE NAITO.** Biocompatibility of Dialytic Membrane. Tokyo Igakusha Ltd, 25 March 2010